(19) Europäisches Patentamt
European Patent Office
Office européen des brevets

(11) **EP 4 497 375 A1**

(12) **EUROPEAN PATENT APPLICATION**
published in accordance with Art. 153(4) EPC

(43) Date of publication:
**29.01.2025 Bulletin 2025/05**

(21) Application number: **23775270.4**

(22) Date of filing: **21.03.2023**

(51) International Patent Classification (IPC):
**A61B 5/00** (2006.01)    **A61B 5/024** (2006.01)
**G16H 50/20** (2018.01)

(52) Cooperative Patent Classification (CPC):
**A61B 5/00; A61B 5/024; G16H 50/20**

(86) International application number:
**PCT/KR2023/003723**

(87) International publication number:
**WO 2023/182774 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.03.2022   KR 20220034751
13.06.2022   KR 20220071467**

(71) Applicant: **Thyroscope Inc.**
**Ulsan 44538 (KR)**

(72) Inventors:
• **PARK, Jaemin**
  **Busan 46279 (KR)**
• **SHIN, Kyubo**
  **Ulju-gun Ulsan 44951 (KR)**

(74) Representative: **Dantz, Jan Henning et al**
**Loesenbeck - Specht - Dantz
Patent- und Rechtsanwälte
Am Zwinger 2
33602 Bielefeld (DE)**

(54) **METHOD FOR MONITORING USER DISEASE ON BASIS OF HEART RATE INFORMATION, AND SERVER PERFORMING SAME**

(57)    Proposed is a method of monitoring diseases of a user on the basis of heart rate information obtained by a wearable device. The method includes obtaining type information of the wearable device and the heart rate information, generating disease monitoring information using the heart rate information through a disease monitoring module when the type information corresponds to a reference type, generating standardized heart rate information using the heart rate information through a heart rate information standardization module and generating the disease monitoring information using the standardized heart rate information through the disease monitoring module when the type information does not correspond to the reference type, and outputting the disease monitoring information.

FIG. 11

OBTAINING TYPE INFORMATION OF WEARABLE DEVICE AND HEART RATE INFORMATION — S1110

GENERATING STANDARDIZED HEART RATE INFORMATION USING TYPE INFORMATION AND HEART RATE INFORMATION THROUGH HEART RATE INFORMATION STANDARDIZATION MODULE — S1120

GENERATING DISEASE MONITORING INFORMATION USING STANDARDIZED HEART RATE INFORMATION THROUGH THE DISEASE MONITORING MODULE — S1130

OUTPUTTING DISEASE MONITORING INFORMATION — S1140

EP 4 497 375 A1

**Description**

BACKGROUND OF THE INVENTION

Field of the Invention

The present disclosure relates to a method of monitoring a disease of a patient using standardized heart rate information, and a server for performing the method.

Description of the Related Art

Technologies that monitor diseases using wearable devices are being developed with the development of wearable devices and medical techniques.

However, since several manufacturers release wearable devices of various models, the kinds of sensors in the wearable devices are different or the processing types of sensing data are different, so there is a problem that different results are derived from the same states when monitoring diseases, depending on wearable device, and as a result, it is difficult to provide disease monitoring with high accuracy.

Further, since even one manufacture releases wearable devices of various versions, the kinds of sensors are different or the processing types of sensing data are different in the versions of those wearable devices, so there may be a problem that different results are derived from the same states when monitoring diseases.

Accordingly, it is required to develop disease monitoring that is compatible for various types of wearable devices.

SUMMARY OF THE INVENTION

An objective of the present disclosure is to accurately monitor a disease of a user regardless of the types of wearable devices.

Objectives of the present disclosure are not limited to those described above and objectives not stated above will be clearly understood to those skilled in the art from the specification.

According to an embodiment, there may be provided a method of monitoring diseases of a user on the basis of heart rate information obtained by a wearable device, the method including: obtaining type information of the wearable device and the heart rate information; generating disease monitoring information using the heart rate information through a disease monitoring module when the type information corresponds to a reference type; generating standardized heart rate information using the heart rate information through a heart rate information standardization module and generating the disease monitoring information using the standardized heart rate information through the disease monitoring module when the type information does not correspond to the reference type; and outputting the disease monitoring information.

The heart rate information standardization module may be implemented using a heart rate information standardization model, and the heart rate information standardization model may be a model trained using training heart rate information and reference heart rate information corresponding to the training heart rate information, the training heart rate information may be heart rate information obtained by a wearable device that is not the reference type for a first time, and the reference heart rate information may be heart rate information obtained by a wearable device that is the reference type for the first time.

The disease monitoring module may be implemented using a first disease monitoring model, the generating of the disease monitoring information using the heart rate information may include generating the disease monitoring information by inputting the heart rate information into the first disease monitoring model, and the generating of the disease monitoring information using the standardized heart rate information may include generating the disease monitoring information by inputting the standardized heart rate information into the first disease monitoring model.

The disease monitoring module may be implemented using a second disease monitoring model, the generating of the disease monitoring information using the heart rate information may include: generating a first rest period heart rate from the heart rate information; and generating the disease monitoring information by inputting the first rest period heart rate into the second disease monitoring model, and the generating of the disease monitoring information using the standardized heart rate information may include: generating a second rest period heart rate from the standardized heart rate information; and generating the disease monitoring information by inputting the second rest period heart rate into the second disease monitoring model.

The disease monitoring module may be implemented further using a rest period heart rate generation model, and the first rest period heart rate and the second rest period heart rate may be generated by inputting the heart rate information and the standardized heart rate information into the rest period heart rate generation model, respectively.

The heart rate information may include a plurality of heart rates corresponding to different points in time, and the standardized heart rate information may include a plurality of standardized heart rates corresponding to different points in time.

The method may further include generating one or more heart rate missing value from the plurality of heart rates, thereby generating missing value estimation heart rate information including the plurality of heart rates and the heart rate missing value, and the standardized heart rate information may be generated through the heart rate information standardization module using the missing value estimation heart rate information.

The heart rate information standardization module may be implemented using a heart rate information standardization model, and the standardized heart rate information may be generated by inputting the heart rate information into the heart rate information standardization model.

The heart rate information standardization module may be implemented using a first type model and a second type model, the standardized heart rate information may be generated using the heart rate information and the first type model when the type information corresponds to a first type that is not the reference type, and the standardized heart rate information may be generated using the heart rate information and the second type model when the type information corresponds to a second type that is not the reference type.

The heart rate information standardization module may be implemented using a sleep model or a non-sleep model, the standardized heart rate information may be generated using heart rate information obtained in sleep and the sleep model when the heart rate information is the heart rate information obtained in sleep, and the standardized heart rate information may be generated using heart rate information obtained in non-sleep and the non-sleep model when the heart rate information is the heart rate information obtained in non-sleep.

According to an embodiment, there may be provided a method of monitoring diseases of a user on the basis of heart rate information obtained by a wearable device, the method including: obtaining type information of the wearable device and the heart rate information; generating standardized heart rate information using the type information and the heart rate information through a heart rate information standardization module; generating disease monitoring information using the standardized heart rate information through a disease monitoring module; and outputting the disease monitoring information.

The heart rate information standardization module may be implemented using a heart rate information standardization model, the heart rate information standardization model may be a model trained using training heart rate information and reference heart rate information corresponding to the training heart rate information, the training heart rate information may be heart rate information obtained by a wearable device for a first time, and the reference heart rate information may be heart rate information obtained by a heart rate information measurement device for the first time.

The disease monitoring module may be implemented using a first disease monitoring model, and the generating of the disease monitoring information may include generating the disease monitoring information by inputting the standardized heart rate information into the first disease monitoring model.

The disease monitoring module may be implemented using a second disease monitoring model, and the generating of the disease monitoring information may include: generating a rest period heart rate from the standardized heart rate information; and generating the disease monitoring information by inputting the rest period heart rate into the second disease monitoring model.

The disease monitoring module may be implemented further using a rest period heart rate generation model, and the rest period heart rate may be generated by inputting the standardized heart rate information into the rest period heart rate generation model.

The heart rate information may include a plurality of heart rates corresponding to different points in time, and the standardized heart rate information may include a plurality of standardized heart rates corresponding to different points in time.

The method may further include generating one or more heart rate missing value from the plurality of heart rates, thereby generating missing value estimation heart rate information including the plurality of heart rates and the heart rate missing value, and the standardized heart rate information may be generated through the heart rate information standardization model using the missing value estimation heart rate information.

The heart rate information standardization module may be implemented using a heart rate information standardization model, and the generating of the standardized heart rate information may include generating the standardized heart rate information by inputting the type information and the heart rate information into the heart rate information standardization model.

The heart rate information standardization module may be implemented using a first type model and a second type model, the standardized heart rate information may be generated using the heart rate information and the first type model when the type information corresponds to a first type, and the standardized heart rate information may be generated using the heart rate information and the second type model when the type information corresponds to a second type.

According to an embodiment, there may be provided a non-transitory computer-readable medium that stores one or more instructions, wherein when the one or more instructions are executed by one or more processors of a device/server, the one or more instructions make the device/server perform the method according to an embodiment.

According to an embodiment, there may be provided a server for monitoring diseases of a user on the basis of heart rate information obtained by a wearable device, the server including: a communication unit; a storage configured to store one or more instructions; and a controller configured to execute the one or more instructions stored in the storage, in which the controller executes the one or more instructions, thereby obtaining type information of the wearable device and the heart rate information; generating disease monitoring information using the heart rate information through a disease monitoring module when the type information corresponds to a reference type; generating standardized heart rate information using the heart rate information through a heart rate information standardization module and generating the disease monitoring information using the standardized heart rate information through the disease monitoring module when the type information does not correspond to the reference type; and outputting the disease monitoring information.

Solutions of the present disclosure are not limited to those described above and solutions not stated above will be clearly understood to those skilled in the art from the specification.

According to an embodiment, information obtained by a wearable device is standardized in consideration of type information of the wearable device and diseases of a user are monitored using the standardized information, whereby it is possible to accurately monitor disease of the user regardless of the types of wearable devices.

Effects of the present disclosure are not limited to the effect described above and effects not stated above will be clearly understood to those skilled in the art from the specification.

BRIEF DESCRIPTION OF THE DRAWINGS

The above and other objectives, features and other advantages of the present invention will be more clearly understood from the following detailed description when taken in conjunction with the accompanying drawings, in which:

FIG. 1 is an example of differences in heart rate information measurement data according to wearable devices;
FIG. 2 is a view showing a disease monitoring system according to an embodiment;
FIG. 3 is a view showing a disease monitoring server according to an embodiment;
FIG. 4 is a flowchart showing a disease monitoring method according to an embodiment;
FIGS. 5A and 5B are views showing generating standardized heart rate information using a heart rate information standardization model according to an embodiment;
FIG. 6 is a view showing generating standardized heart rate information using a plurality of type models model according to an embodiment;
FIG. 7 is a view showing generating standardized heart rate information using a sleep model and a non-sleep model according to an embodiment;
FIGS. 8A and 8B are views showing generating disease monitoring information using a disease monitoring model according to an embodiment;
FIG. 9 is a view showing obtaining a representative heart rate according to an embodiment;
FIGS. 10A and 10B are views showing generating standardized heart rate information using heart rate information having a missing value according to an embodiment;
FIG. 11 is a flowchart showing a disease monitoring method according to another embodiment;
FIG. 12 is a flowchart showing a first example of performing a disease monitoring method in a disease monitoring system according to an embodiment;
FIG. 13 is a flowchart showing a second example of performing a disease monitoring method in a disease monitoring system according to an embodiment;
FIG. 14 is a flowchart showing a third example of performing a disease monitoring method in a disease monitoring system according to an embodiment;
FIG. 15 is a flowchart showing a fourth example of performing a disease monitoring method in a disease monitoring system according to an embodiment;
FIG. 16 is a flowchart showing a user registration step according to an embodiment;
FIG. 17 is a graph showing the result of measuring heart rate information of a specific user through an Apple Watch and a Fitbit;
FIG. 18 is a graph showing standardizing heart rate information in accordance with the type information of a wearable device when Fitbit is a reference type;
FIG. 19 is a graph showing standardizing heart rate information in accordance with the type information of a wearable device when Apple Watch is a reference type; and
FIG. 20 is a graph showing the standardized heart rate information of both FIG. 18 and FIG. 19.

DETAILED DESCRIPTION OF THE INVENTION

Embodiments described herein are provided to clearly explain the spirit of the present disclosure to those skilled in the art, so the present disclosure is not limited to the embodiments described herein and the scope of the present disclosure should be construed as including changed or modified examples not departing from the spirit of the present disclosure.

Terminologies used herein were selected from general terminologies that are used at present as generally as possible in consideration of their functions herein, but may be changed, depending on the intention of those skilled in the art, customs, advent of new technologies, or the like. However, when such specific terminologies are defined and used as certain meanings, the meanings of the terminologies will be specifically described. Accordingly, the terminologies used herein should be construed on the basis of the substantial meanings of the terminologies and the entire specification, not simply the names of the terminologies.

Drawings of this specification are provided to easily explain the present disclosure and the shapes shown in the drawings may be exaggerated, if necessary, to help understand the present disclosure, so the present disclosure is not limited to the drawings.

When it is determined that detailed description of well-known configurations or functions related to the present disclosure may make the spirit of the present disclosure unclear, they are not described in detail, if necessary. Further, numbers (e.g., first, second, etc.) used in the description of the present disclosure, unless specifically stated, are only identification symbols to discriminate one component from another component.

Singular forms are intended to include plural forms unless the context clearly indicates otherwise. It will be further understood that the terms "comprise" or "composed of" used in this specification, specify the presence of stated features, steps, operations, components, parts, or a combination thereof, but do not preclude the presence or addition of one or more other features, numerals, steps, operations, components, parts, or a combination thereof.

Hereafter, a method of monitoring a disease of a user according to the present disclosure, and a server and system using the method are described. For the convenience of description, monitoring thyroid dysfunction (e.g., hyperthyroidism and hypothyroidism) that is a thyroid disease is mainly described, but disease monitoring according to the present disclosure is not limited thereto.

In the specification, heart rate information may mean a set of heart rate values. For example, the heart rate information may mean a set of heart rate values measured for a predetermined time.

In the specification, standardization of heart rate information means operation or a step that is performed to reduce a deviation or an error between measurement data obtained from devices measuring heart rate information.

In the specification, standardized heart rate information means heart rate information generated by standardizing heart rate information. In this case, standardization may mean standardizing according to an embodiment of the specification. In other words, when it is stated simply as heart rate information, it may mean heart rate information when standardization according to an embodiment of the specification is not performed or before standardization according to an embodiment of the specification is performed.

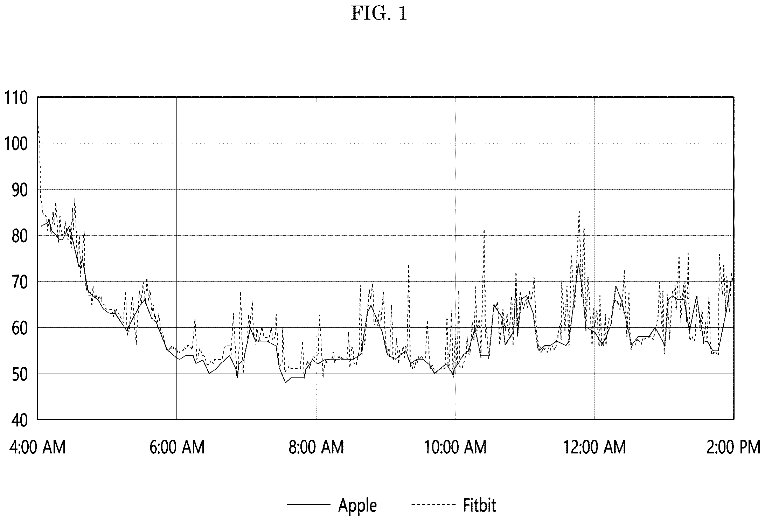

FIG. 1, which is an example of differences in heart rate information measurement data according to wearable devices, is a graph showing the result of measuring a heart rate of a user with Apple Watch and Fitbit on the same wrist. The x axis represents time and the y axis represents heart rate in FIG. 1.

Referring to FIG. 1, it is possible to see that the heart rate measured by Apple Watch and the heart rate measured by Fitbit are different even at the same points in time. A problem that can be derived from this fact is that a result of performing disease monitoring on the basis of the heart rate measured by Apple Watch and a result of performing disease monitoring on the basis of the heart rate measured by Fitbit may be different, and accordingly, this may influence the accuracy in a disease monitoring result.

Further, Fitbit measures a heart rate once for every one minute, but Apple Watch irregularly measures a heart rate once for every 5~8 minutes. In other words, different heart rates may be measured even at the same point in time and the points in time at which measuring a heart rate may be different, depending on wearable devices.

Accordingly, a disease monitoring method that can be applied to various types of wearable devices is required.

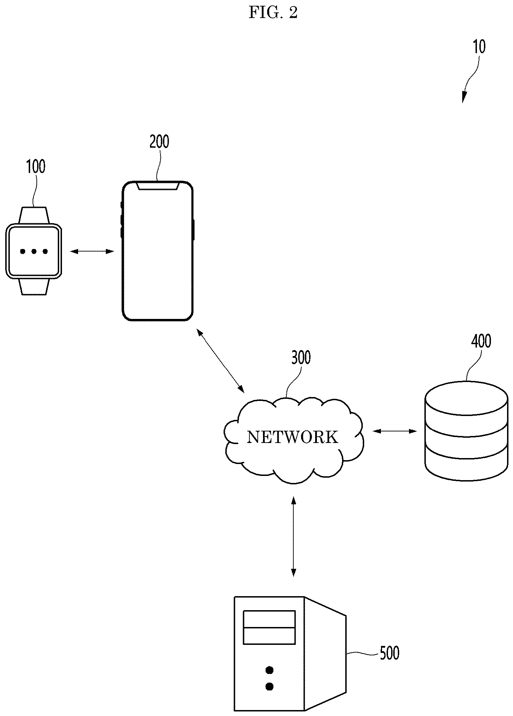

FIG. 2 is a view showing a disease monitoring system 10 according to an embodiment.

Referring to FIG. 2, a network 300, for example, can connect any two or more of a use terminal 200, a data storage server 400, and a disease monitoring server 500 such that communication is possible (directly or indirectly).

In FIG. 2, a wearable device 100 is not directly connected to the network 300 and is connected to the network 300 through the user terminal 200, but in some embodiments, the wearable device 100 may be directly connected to the network 300.

The wearable device 100 can sense heart rate information of a user. The wearable device 100 can obtain heart rate information on the body of a user. In this case, the user may mean a person wearing the wearable device 100.

The wearable device 100 may be, for example, a smart watch, a smart band, a smart ring, of a combination thereof, but is not limited thereto.

The user terminal 200 can obtain heart rate information from the wearable device 100. The user terminal 200 can provide heart rate information to the data storage server 400.

The user terminal 200 may be, for example, a smartphone, a tablet device, a laptop, a personal computer, or a combination thereof, but is not limited thereto.

The data storage server 400 can store heart rate information. For example, the data storage server 400 can store heart rate information into a heart rate information database as a database.

The data storage server 400, for example, may be a server that a wearable device manufacturer operates or manages, but is not limited thereto.

The disease monitoring server 500 can monitor diseases using heart rate information. The disease monitoring server 500 can generate disease monitoring information using heart rate information.

The disease monitoring server 500 can standardize heart rate information. The disease monitoring server 500 can generate standardized heart rate information.

Disease monitoring and heart rate information standardization by the disease monitoring server 500 will be described in more detail below.

The components shown in FIG. 2 are not all necessary components of the disease monitoring system 10 and at least some of the components of the disease monitoring system 10 shown in FIG. 2 may be omitted. For example, the disease monitoring system 10 may not include the data storage server 400. In this case, the function of the data storage server 400 may be performed by other components of the disease monitoring system 10 such as the disease monitoring server 500 and the user terminal 200. As another example, the disease monitoring system 10 may not include the user terminal 200. In this case, the function of the user terminal 200 may be performed by other components of the disease monitoring system 10 such as the wearable device 100. In addition, the disease monitoring system 10 may further include components not shown in FIG. 2.

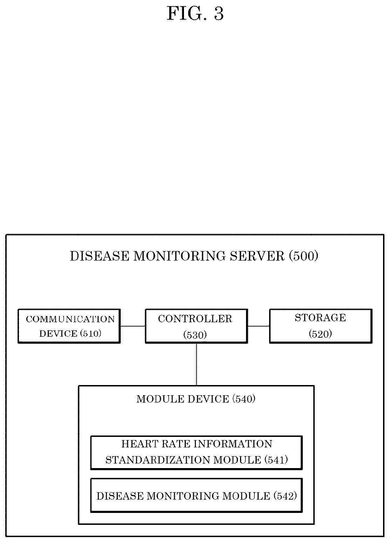

FIG. 3 is a view showing the disease monitoring server 500 according to an embodiment. Referring to FIG. 3, the disease monitoring server 500 may include a communication unit 510, a storage 520, a controller 530, and a module unit 530.

The disease monitoring server 500 can perform communication with the outside through the communication unit 510. For example, the disease monitoring server 500 can receive heart rate information from the data storage server 400 through the communication unit 510. As another example, the disease monitoring server 500 can transmit disease monitoring information to the user terminal 200 through the communication unit 510.

The communication unit 510 can perform wired or wireless communication. The communication unit 510 may be, for example, a wired/wireless Local Area Network (LAN) module, a WAN module, an Ethernet module, a Bluetooth module, a Zigbee module, a Universal Serial Bus (USB) module, an IEEE 1394 module, a WiFi module, or a combination thereof, but is not limited thereto.

The disease monitoring server 500 can store various data, programs, applications, or the like for operation in the storage 520. The programs or applications stored in the storage 520 may include one or more instructions. The programs or applications stored in the storage 520 can be executed by the controller 530.

The storage 520 can store information that the disease monitoring server 500 obtains. For example, the storage 520 can store heart rate information, user information, and type information of wearable devices. The storage 520 can store the result of operation that the disease monitoring server 500 performs, etc. For example, the storage 520 can store disease monitoring information.

The storage 520, for example, may be a hard disk, a flash memory, a Solid State Drive (SSD), a Random Access Memory (RAM), a Read Only Memory (ROM), an Electrically Erasable Programmable Read-Only Memory (EEPROM), a magnetic memory, a magnetic disc, an optical disc, or a combination thereof, but is not limited thereto.

The disease monitoring server 500 can process various items of information and can perform operation through the controller 530. For example, the controller 530 can generate standardized heart rate information. As another example, the controller 530 can generate disease monitoring information. As another example, the controller 530 can transmit standardized heart rate information to an external device or an external server, generate disease monitoring information on the basis of the standardized heart rate information at the external device or external server, and receive the generated disease monitoring information from the external device or external server. The controller 530 can control other components constituting the disease monitoring server 500.

The controller 530 may be implemented as a computer or a similar device in accordance with hardware, software, or a combination thereof. As hardware, the controller 530 may be one or a plurality of processors. Alternatively, the controller 530 may be provided as processors that are physically spaced apart and cooperate through communication. The controller 530 may be, for example, a Central Processing Unit (CPU), a Graphics Processing Unit (GPU), a Digital Signal Processor (DSP), a state machine, an Application Specific Integrated Circuit (ASIC), a Radio-Frequency Integrated Circuit (RFIC), or a combination thereof, but is not limited thereto.

As software, the controller 530 may be provided in the form of a program that drives the controller 530 that is hardware.

Unless specifically state hereafter, the operation of the disease monitoring server 500 may be construed as being performed by the controller 530 or performed by control of the controller 530.

The module unit 540 may include one or more operable modules. The module unit 540 may include a heart rate information standardization module 541. The heart rate information standardization module 541 may be implemented using one or more models. The module unit 540 may include a disease monitoring module 542. The disease monitoring module 542 may be implemented using one or more models. The module unit 540 will be described in more detail below.

The components shown in FIG. 3 are not all necessary components of the disease monitoring server 500 and at least some of the components of the disease monitoring server 500 shown in FIG. 3 may be omitted. In addition, the disease monitoring server 500 may further include components not shown in FIG. 3. For example, the disease monitoring server 500 may further include an output device including a display, a speaker, or a combination thereof.

Although one or more operable modules are all included in the module unit 540 in FIG. 3, at least one or more modules of the modules may be implemented as software modules stored in the storage 520. For example, the heart rate information standardization module 541 may be stored in the storage 520 as a software module without being implemented as a specific hardware module included in the module unit 540, as shown in FIG. 3, and may be operated by being executed by the controller 530. As another example, the disease monitoring module 542 may be stored in the storage 520 as a software module without being implemented as a specific hardware module included in the module unit 540, as shown in FIG. 3, and may be operated by being executed by the controller 530.

Hereafter, a disease monitoring method according to the present disclosure is described. A disease monitoring method is performed at a disease monitoring server in the following description, but the disease monitoring method is not necessarily performed at a disease monitoring server. For example, the disease monitoring method may be performed at a user terminal. As another example, a portion of the disease monitoring method may be performed at a disease monitoring server and the other may be performed at a user terminal.

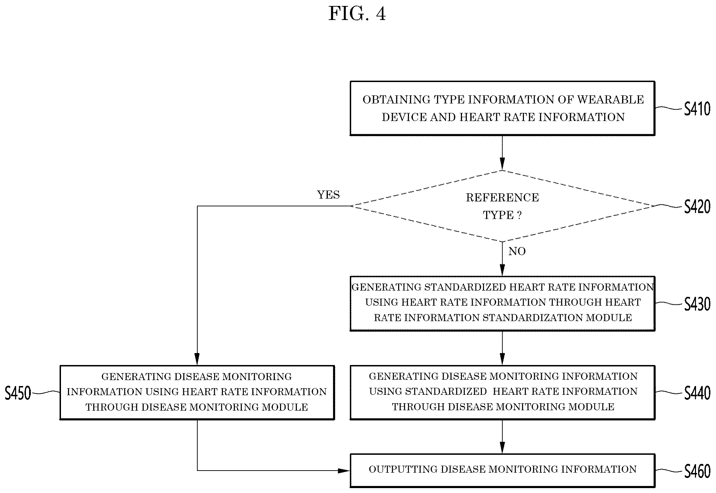

FIG. 4 is a flow chart showing a disease monitoring method according to an embodiment.

In step S410, a disease monitoring server can obtain type information of a wearable device and heart rate information.

According to an embodiment, the types of wearable devices may correspond to the manufacturers of the wearable devices. For example, wearable devices manufactured by Apple may correspond to an apple type, wearable devices manufactured by Fitbit may correspond to a Fitbit type, and wearable devices manufactured by Samsung Electronics may correspond to a Samsung Electronics type. In this case, when wearable devices are manufactured by the same manufacturer, they may correspond to the same type even though the models thereof are different. For example, the Apple Watch 1st to 7th generations may correspond to the same apple type and the Galaxy Watch 1st to 3rd generations may correspond to the same Samsung electronics type.

In this case, different items of type information may be set in accordance with the manufacturers of wearable devices. The type information may be manually set by a user or automatically set by a disease monitoring server.

According to an embodiment, the types of wearable devices may correspond to the models of the wearable devices. In this case, when wearable devices of different models manufactured by the same manufacturers may correspond to different types, respectively. For example, even though wearable devices are wearable devices manufactured by Apple, the Apple Watch 1st to 7th generations may correspond to different types, respectively. As another example, some of the Apple Watch 1st to 7th generations may correspond to types different from those of the others.

In this case, different items of type information may be set in accordance with the models of wearable devices. The type information may be manually set by a user or automatically set by a disease monitoring server.

According to another embodiment, the types of wearable devices may be discriminated in accordance with identity of measurement values that are measured by the wearable devices. For example, when a first wearable device and a second wearable device provide the same measurement values (e.g., heart rate information, etc.), the first wearable device and the second wearable device may correspond to the same type.

In this case, different items of type information may be set in accordance with the identity of the measurement values. The type information may be manually set by a user or automatically set by a disease monitoring server.

A disease monitoring server can obtain type information through some methods.

According to an embodiment, the disease monitoring server can receive a user identifier from at least one of a user terminal, a data storage server, and a wearable device and can determine type information on the basis of the user identifier. For example, the disease monitoring server can store in advance a user identifier and type information corresponding to the user identifier, and can determine type information by finding an identifier that is the same as the received identifier from pre-stored identifiers.

According to another embodiment, the disease monitoring server can receive type information from at least one of a user terminal, a data storage server, and a wearable device.

The disease monitoring server can obtain heart rate information from at least one of the user terminal, the data storage server, and the wearable device.

For example, the disease monitoring server can obtain heart rate information from the data storage server. In this case, the disease monitoring server may receive a user identifier or type information from the data storage server, but may receive a user identifier or type information from the user terminal or the wearable device.

As another example, the disease monitoring server can obtain heart rate information from the user terminal. In this case, the disease monitoring server may receive a user identifier or type information from the user terminal, but may receive a user identifier or type information from the data storage server or the wearable device.

The disease monitoring server can obtain type information and heart rate information in the same cycle.

Alternatively, the disease monitoring server can obtain type information and heart rate information in different cycles. For example, the disease monitoring server can obtain heart rate information in a longer cycle than type information. As a more detailed example, the disease monitoring server can obtain type information in a first cycle and heart rate information in a second cycle longer than the first cycle. As another example, the disease monitoring server can obtain type information in a longer cycle than heart rate information. As a more detailed example, the disease monitoring server can obtain heart rate information in a first cycle and type information in a second cycle longer than the first cycle.

The disease monitoring server can obtain type information and heart rate information in the same number of times. For example, the disease monitoring server can obtain hear rate information one time when obtaining type information one time.

Alternatively, the disease monitoring server can obtain type information and heart rate information in different number of times. For example, the disease monitoring server can obtain heart rate information in a larger number of times than type information. As a more detailed example, the disease monitoring server can obtain hear rate information two or more times when obtaining type information one time. As another example, the disease monitoring server can obtain type information in a larger number of times than heart rate information. As a more detailed example, the disease monitoring server can obtain type information two or more times when obtaining heart rate information one time.

In step S420, in some embodiments, the disease monitoring server can determine whether type information corresponds to a reference type. The reference type may be a specific type of the types of a plurality of wearable devices.

In step S430, when type information does not correspond to a reference type, the disease monitoring server can generate standardized heart rate information using heart rate information through the heart rate information standardization module.

Generating standardized heart rate information using heart rate information may means converting heart rate information obtained from a non-reference type wearable device into heart rate information obtained from a reference type wearable device. For example, when a Fitbit type is a reference type, the disease monitoring server can convert heart rate information obtained from another type wearable device (e.g., Apple Watch, Galaxy Watch, etc.) that is not Fitbit into heart rate information obtained from Fitbit. As another example, when a first model of Fitbit is a reference type, the disease monitoring server can convert heart rate information obtained from a second model (different from the first model) of Fitbit into heart rate information obtained from the first model of Fitbit. In other words, standardized heart rate information may be a virtual heart rate information that is expected as one obtained from a reference type wearable device.

For example, heart rate information may include a plurality of heart rate values and the points in time at which the heart rate values were obtained. As a more detailed example, heart rate information may be expressed as an n×2 matrix.

$$\begin{pmatrix} first\ point\ in\ time & first\ heart\ rate \\ second\ point\ in\ time & second\ heart\ rate \\ \cdot & \cdot \\ \cdot & \cdot \\ \cdot & \cdot \\ n-th\ point\ in\ time & n-th\ heart\ rate \end{pmatrix}$$

where n is the number of heart rates, the first point in time is the earliest point in time at which a heart rate was measured, and the n-th point in time is the latest point in time at which a heart rate was measured.

In some embodiments, the interval between points in time at which heart rate was measured may be a predetermined time interval. For example, the interval may be 10 seconds, 30 second, 1 minute, 5 minutes, or 10 minutes, but is not limited thereto.

As another example, heart rate information may include a plurality of heart rate values. As a more detailed example, heart rate information may be expressed as the following n-order vector.

$$\begin{pmatrix} first\ heart\ rate \\ second\ heart\ rate \\ \cdot \\ \cdot \\ \cdot \\ n-th\ heart\ rate \end{pmatrix}$$

where n is the number of heart rates.

As another example, heart rate information may include electrocardiogram (ECG) data. As a more detailed example, heart rate information may include at least one lead ECG data, but is not limited thereto.

As another example, heart rate information may include a plurality of ECG values and the points in time at which the ECG values were measured. As a more detailed example, heart rate information may include time series data including a plurality of ECG values and the points in time at which the ECG values were measured, but is not limited thereto.

According to an embodiment, the heart rate information standardization module may be implemented using a heart rate information standardization model.

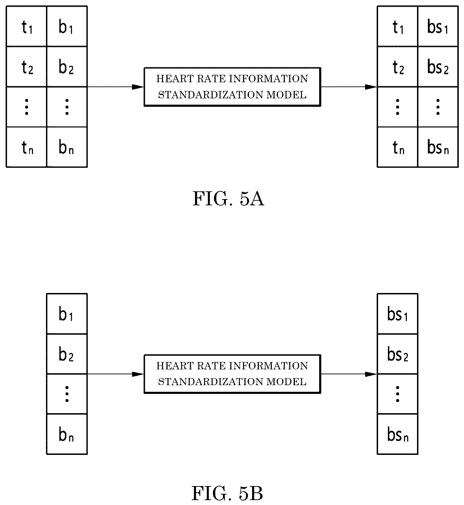

FIGS. 5A and 5B show generating standardized heart rate information using a heart rate information standardization model, in which FIG. 5A is the case in which standardized heart rate information is generated from heart information of the n×2 matrix type heart rate information including heart rate values and points in time described above and FIG. 5B is the case in which standardized heart rate information is generated from heart rate information of the n-order vector type including heart rate values described above.

The disease monitoring server can generate standardized heart rate information using heart rate information and a heart rate information standardization model. For example, the disease monitoring server can generate standardized heart rate information by inputting heart rate information into a heart rate information standardization model. As another example, the disease monitoring server can generate standardized heart rate information by inputting information generated from heart rate information into a heart rate information standardization model. The information generated from heart rate information may be, for example, information in which at least some heart rate values included in the heart rate information have been changed or deleted, information generated by adding one or more value to the heart rate information, or information generated by processing the heart rate information, but is not limited thereto.

For example, referring to FIG. 5A, heart rate information may include a plurality of heart rate values b1, b2, to bn and measurement points in time t1, t2, to tn at which the heart rate values were obtained, and standardized heart rate information may include a plurality of standardized heart rate values bs1, bs2, to bsn and points in time t1, t2, to tn corresponding to the standardized heart rate values. In this case, the points in time corresponding to the standardized heart rate values may be the same as the measurement points in time at which the heart rate values were obtained. Standardized heart rate information includes points in time t1, t2, to tn corresponding to the standardized heart rate values in FIG. 5A, but the standardized heart rate information may include standardized heart rate values bst, bst, to bsn without including the points in time t1, t2, to tn corresponding to the standardized heart rate values.

As another example, referring to FIG. 5B, heart rate information may include a plurality of heart rate values b1, b2, to bn and standardized heart rate information may include a plurality of standardized heart rate values bs1, bs2, to bsn.

A heart rate information standardization model may be a machine learning model, a deep learning model, or a combination thereof. The heart rate information standardization model may be, for example, an RNN-series model such as an RNN model, an LSTM model, or a GRU model, but is not limited thereto.

The heart rate information standardization model may include a plurality of layers. For example, the heart rate information standardization model may include an input layer, a hidden layer, and an output layer. The input layer may be a layer in which heart rate information is input. The output layer may be a layer in which heart rate information is output. The hidden layer may be a layer between the input layer and the output layer.

The heart rate information standardization model can be trained through various methods such as supervised learning, unsupervised learning, semi-supervised learning, and reinforcement learning.

For example, the heart rate information standardization model may be trained through supervised learning. In this case, the heart rate information standardization model can be trained using training heart rate information and reference heart rate information corresponding to the heart rate information for learning. As a more detailed example, the heart rate information standardization model can receive training heart rate information, can output output heart rate information, and can be trained on the basis of the difference between the output heart rate information and reference heart rate information. The heart rate information for learning may be heart rate information obtained from a non-reference type wearable device for a predetermined time. The reference heart rate information may be heart rate information obtained from a reference type wearable device for a predetermined time. Alternatively, the reference heart rate information may be heart rate information expected as one obtained from a reference type wearable device for a predetermined time. Selectively, the heart rate information standardization model may be trained further using type information. In this case, the heart rate information standardization model can receive training heart rate information and type information, can output heart rate information, and can be trained on the basis of the difference between the output heart rate information and reference heart rate information.

Selectively, the heart rate information standardization model may be trained further using biological information such as the sex and age of a person.

The disease monitoring server can train the heart rate information standardization model. Alternatively, the disease monitoring server can be provided with a trained heart rate information standardization model from the outside.

Selectively, the disease monitoring server can generate standardized heart rate information further using type information. For example, the disease monitoring server can generate standardized heart rate information by inputting heart rate information and type information into the heart rate information standardization model. When the heart rate information standardization model includes an input layer, a hidden layer, and an output layer, heart rate information and type information both may be input to the input layer. Alternatively, when the heart rate information standardization model includes an input layer, a hidden layer, and an output layer, heart rate information may be input to the input layer and type information may be output to the hidden layer or the output layer.

According to another embodiment, the heart rate information standardization module may be implemented using a plurality of type models. For example, the heart rate information standardization module may be implemented using at least a first type model and a second type model.

The characteristic or pattern of heart rate information that is measured may depend on the types of wearable devices. For example, the measurement methods of heart rate information of wearable devices may be different, whereby the characteristics or patterns of heart rate information that is measured may be different. Accordingly, when heart rate information obtained from different types of wearable devices is standardized using one same model, incorrect standardized heart rate information may be generated. In this case, it is possible to generate more accurately standardized heart rate information using different type models in accordance with the types of wearable devices.

Further, the matter described through FIGS. 5A and 5B can be applied also to the case in which heart rate information includes the ECG data described above and the case in which heart rate information includes a plurality of ECG values and the points in time at which the ECG values were obtained, so repeated description is omitted.

Further, according to an embodiment, a training data obtaining method for implementing a heart rate information standardization module may be provided.

A training data obtaining method according to an embodiment may include a step of obtaining first type heart rate information and second type heart rate information from a first type wearable device and a second type wearable device that are positioned on the body of the same user.

In this case, the first type wearable device may correspond to a reference type wearable device and the second type wearable device may correspond to a non-reference type wearable device, but they are not limited thereto.

For example, the first type wearable device may correspond to a Fitbit wearable device and the second type wearable device may correspond to an Apple wearable device, but they are not limited thereto.

Further, for example, the first type wearable device may correspond to an Apple 5th generation wearable device and the second type wearable device may correspond to an Apple 6th generation wearable device, but they are not limited thereto.

Further, for example, the first type wearable device may correspond to a wearable device that has been approved in a clinical test and the second type wearable device may correspond to a wearable device that has not been approved in a clinical test yet, but they are not limited thereto. In this case, the meaning of the wearable device that has been approved in a clinical test may mean approval in a clinical test for heart rate itself obtained from the wearable device, but is not limited thereto and may mean approval in a clinical test related to disease information output using heart rate information obtained from the wearable device.

In the step of obtaining first type heart rate information and second type heart rate information from a first type wearable device and a second type wearable device that are positioned on the body of the same user according to an embodiment, the first type wearable device and the second type wearable device may be positioned at adjacent portions on the body of the same user, and may be guided to be positioned.

For example, the first type wearable device and the second type wearable device both may be positioned around the left wrist of the same user, and may be guided to be positioned around the left wrist, but are not limited thereto.

Further, the training data obtaining method according to an embodiment may include a step of obtaining first type heart rate information and second type heart rate information from a heart rate information measurement device and a wearable device that are positioned on the body of the same user.

In this case, the heart rate information measurement device, for example, may a heart rate monitor and may correspond to a reference type device, and the wearable device may correspond to a non-reference type wearable device, but they are not limited thereto.

Further, the training data obtaining method according to an embodiment may include a step of obtaining first type heart rate information and second type heart rate information from a first type ECG measurement wearable device and a second type ECG measurement wearable device that are positioned on the body of the same user.

In this case, the first type ECG measurement wearable device may correspond to a reference type wearable device and the second type ECG measurement wearable device may correspond to a non-reference type wearable device, but they are not limited thereto.

Further, for example, the first type ECG measurement wearable device may correspond to a wearable device that has been approved in a clinical test and the second type ECG measurement wearable device may correspond to a wearable device that has not been approved in a clinical test yet, but they are not limited thereto. In this case, the meaning of the wearable device that has been approved in a clinical test may mean approval in a clinical test for heart rate itself obtained from the wearable device, but is not limited thereto and may mean approval in a clinical test related to disease information output using heart rate information obtained from the wearable device.

Further, the training data obtaining method according to an embodiment may include a step of obtaining first type heart rate information and second type heart rate information from an ECG measurement device and an ECG measurement wearable device that are positioned on the body of a same user.

In this case, ECG measurement device, for example, may include a heart rate monitor and may correspond to a reference type device, and the ECG measurement wearable device may correspond to a non-reference type wearable device, but they are not limited thereto.

The training data obtaining method according to an embodiment may include a step of generating training data using first type heart rate information and second type heart rate information.

In this case, the training data may include training input data and training result data corresponding to the training input data.

According to an embodiment, the training input data may be generated on the basis of second type heart rate information not corresponding to a reference type.

For example, the training input data according to an embodiment may be obtained by processing at least a portion of second type heart rate information, but is not limited thereto.

Further, in this case, the second type heart rate information may be processed to have a reference time length or a reference format, but is not limited thereto.

According to an embodiment, the training result data may be generated on the basis of first type heart rate information corresponding to a reference type.

For example, the training result data according to an embodiment may be obtained by processing at least a portion of first type heart rate information, but is not limited thereto.

Further, in this case, the first type heart rate information may be processed to have a reference time length or a reference format, but is not limited thereto.

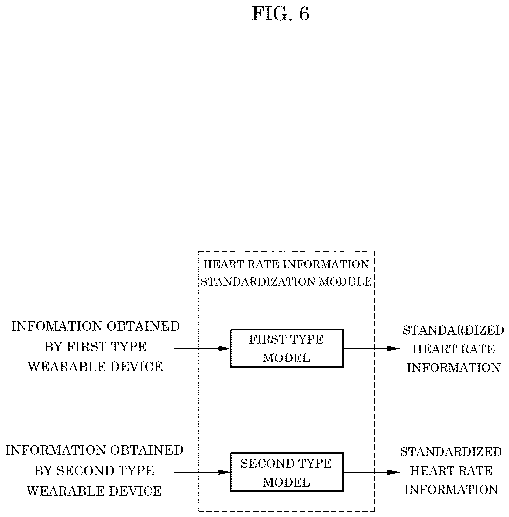

FIG. 6 is a view showing generating standardized heart rate information using a plurality of type models model according to an embodiment.

The disease monitoring server can generate standardized heart rate information using different type models in accordance with the types of wearable devices.

The disease monitoring server can generate standardized heart rate information using heart rate information and a first type model when the type information of a wearable device corresponds to a first type. For example, the disease monitoring server can generate standardized heart rate information by inputting heart rate information into the first type model when the type information of a wearable device corresponds to the first type. As another example, the disease monitoring server can generate standardized heart rate information by inputting information generated from heart rate information into the first type model when the type information of a wearable device corresponds to the first type.

The disease monitoring server can generate standardized heart rate information using heart rate information and a second type model when the type information of a wearable device corresponds to a second type. For example, the disease monitoring server can generate standardized heart rate information by inputting heart rate information into the second type model when the type information of a wearable device corresponds to the second type. As another example, the disease monitoring server can generate standardized heart rate information by inputting information generated from heart rate information into the second type model when the type information of a wearable device corresponds to the second type. In this case, the second type is a type different from the first type.

The matter described with reference to FIGS. 5A and 5B can be similarly applied to the heart rate information and standardized heart rate information in FIG. 6, so repeated description is omitted.

A type model may be a machine learning model, a deep learning model, or a combination thereof. The type model may be, for example, an RNN-series model such as an RNN model, an LSTM model, or a GRU model, but is not limited thereto.

The type model can be trained through various methods such as supervised learning, unsupervised learning, semi-supervised learning, and reinforcement learning.

The matter about training the heart rate information standardization model described above can be similarly applied to training a type model. However, training heart rate information for a type model may include only heart rate information obtained by a wearable device corresponding to the type model. For example, training heart rate information for a first type model may be heart rate information obtained by a first type wearable device and training heart rate information for a second type model may be heart rate information obtained by a second type wearable device.

The disease monitoring server can train a type model. Alternatively, the disease monitoring server can be provided with a trained type model from the outside.

According to another embodiment, the heart rate information standardization module may be implemented using a sleep model or a non-sleep model.

Since heart rate information in sleep is heart rate information that is stable in comparison to heart rate information in non-sleep, the characteristic or pattern of the heart rate information in sleep may be different from those in non-sleep. Accordingly, when heart rate information in sleep and heart rate information in non-sleep are standardized using one same model, incorrect standardized heart rate information may be generated. In this case, when different models are used for the heart rate information in sleep and the heart rate information in non-sleep, it is possible to more accurately generate standardized heart rate information.

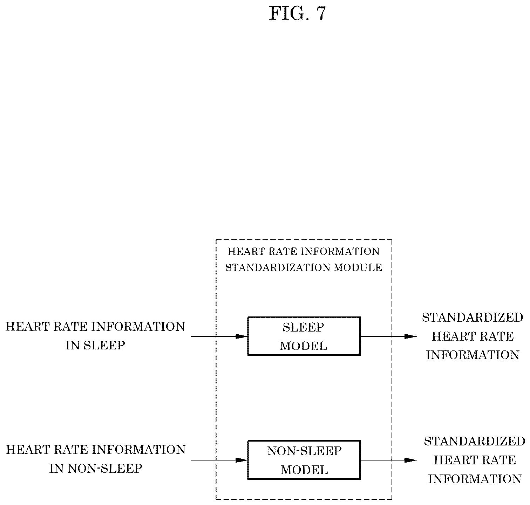

FIG. 7 is a view showing generating standardized heart rate information using a sleep model and a non-sleep model according to an embodiment.

The disease monitoring server can generate standardized heart rate information using different models, depending on whether heart rate information was obtained in sleep or non-sleep.

The disease monitoring server can generate standardized heart rate information using heart rate obtained in sleep and a sleep model. For example, the disease monitoring server can generate standardized heart rate information by inputting heart rate information obtained in sleep or information generated from the heart rate information into a sleep model.

The disease monitoring server can generate standardized heart rate information using heart rate obtained in non-sleep and a non-sleep model. For example, the disease monitoring server can generate standardized heart rate information by inputting heart rate information obtained in non-sleep or information generated from the heart rate information into a non-sleep model.

Selectively, the disease monitoring server can determine whether heart rate information corresponds to the case of sleep or non-sleep. For example, the disease monitoring server can determine whether heart rate information corresponds to the case of sleep or non-sleep by analyzing the heart rate information. As another example, the disease monitoring server can determine whether heart rate information corresponds to the case of sleep or non-sleep in accordance with the point in time at which the heart rate information was obtained. As a more detailed example, when the point in time at which heart rate information was obtained is included in a predetermined sleep time (e.g., from 2 a.m. to 6 a.m.), the disease monitoring server can determine that the heart rate information corresponds to the case of sleep.

Selectively, the disease monitoring server can obtain information about whether heart rate information corresponds to the case of sleep or non-sleep from at least one of a user terminal a data storage server, and a wearable device. For example, the disease monitoring server can obtain information about whether hear rate information corresponds to the case of sleep or non-sleep together with the heart rate information when obtaining the heart rate information from at least one of a user terminal, a data storage server, and a wearable device.

The matter described with reference to FIGS. 5A and 5B can be similarly applied to the heart rate information and standardized heart rate information in FIG. 7, so repeated description is omitted.

A sleep model or a non-sleep model may be a machine learning model, a deep learning model, or a combination thereof. The sleep model or the non-sleep model may be, for example, an RNN-series model such as an RNN model, an LSTM model, or a GRU model, but is not limited thereto.

The sleep model or the non-sleep model can be trained through various methods such as supervised learning, unsupervised learning, semi-supervised learning, and reinforcement learning.

The matter about training the heart rate information standardization model described above can be similarly applied to training a sleep model or a non-sleep. However, training heart rate information for a sleep model may be heart rate information obtained in sleep and training heart rate information for a non-sleep model may be heart rate information obtained in non-sleep.

The disease monitoring server can train a sleep model or a non-sleep model. Alternatively, the disease monitoring server can be provided with a trained sleep model or non-sleep model from the outside.

Referring to FIG. 4 again, the disease monitoring server can generate disease monitoring information using heart rate information or standardized heart rate information using the disease monitoring module.

For example, the disease monitoring information may include information about whether there is a disease. The information about whether there is a disease may be, for example, information about whether there is thyroid dysfunction, but is not limited thereto.

As another example, the disease monitoring information may include information about factors related to one or more diseases. The factors may be, for example, hormone values (e.g., the value of at least one of all hormones related to the thyroid gland that are secreted from the thyroid gland or stimulate the thyroid gland such as free T4, T3, and TSH), but are not limited thereto.

As another example, the disease monitoring information may include information about whether there is a disease and information about factors related to one or more diseases.

In step S440, when type information does not correspond to a reference type, the disease monitoring server can generate disease monitoring information using standardized heart rate information through the disease monitoring module.

In step S450, when type information corresponds to a reference type, the disease monitoring server can generate disease monitoring information using heart rate information through the disease monitoring module. In this case, the disease monitoring server can generate disease monitoring information without the heart rate standardization step of step S430. In other words, when type information corresponds to a reference type, hear rate information obtained by a wearable device can be considered as already coinciding with a reference, so the disease monitoring server can generate disease monitoring information without the heart rate information standardization step. For example, when a Fitbit type is a reference type, the disease monitoring server can generate disease monitoring information without the heart rate information standardization step using heart rate information obtained by Fitbit.

According to an embodiment, the disease monitoring module may be implemented using a disease monitoring model.

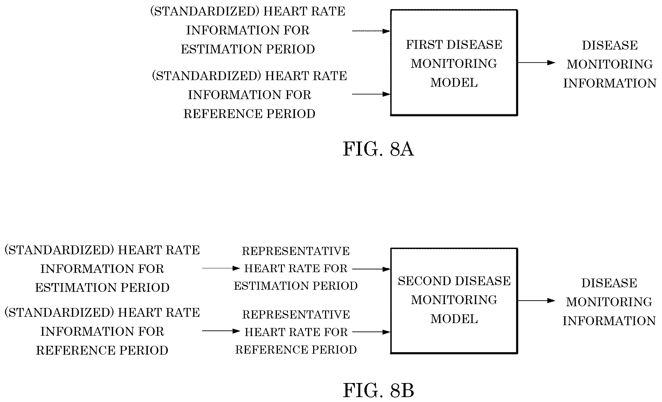

FIGS. 8A and 8B show generating disease monitoring information using a disease monitoring model, in which FIG. 8A is a disease monitoring model (hereafter, referred to as a "first disease monitoring model") that receives heart rate information or standardized heart rate information and outputs disease monitoring information and FIG. 8B is a disease monitoring model (hereafter, referred to as a "second disease monitoring model") that receives one or more representative heart rates generated from heart rate information or standardized heart rate information and outputs disease monitoring information. Hereafter, the "heart rate information or standardized heart rate information" is referred to as "(standardized) heart rate information".

Referring to FIG. 8A, the disease monitoring server according to an embodiment can generate disease monitoring information by inputting (standardized) heart rate information into the first disease monitoring model. In this case, the (standardized) heart rate information that is input into the first disease monitoring model, for example, may be a format including a heart rate value and a point in time, as shown in FIG. 5A, or a format including a heart rate value, as shown in FIG. 5B, but is not limited thereto.

Further, in this case, the (standardized) heart rate information that is input into the first disease monitoring model, for example, may be an ECG data format or a format including an ECG value and the point in time at which the ECG value was obtained, as described with reference to FIGS. 5A and 5B, but is not limited thereto.

The disease monitoring server can generate disease monitoring information by inputting (standardized) heart rate information for an estimation period into the first disease monitoring model.

The estimation period may be a target period for monitoring diseases of a user. For example, the estimation period may be a period including a point in time at which it will be tried to expect whether there is a disease. As another example, the estimation period may be a period including a point in time at which it will be tried to expect factors related to diseases. The estimation period may be the length of a predetermined period. The estimation period may include a plurality of days. The estimation period may be 3 or more days.

Further, the (standardized) heart rate information for the estimation period may mean heart rate information for a partial period included in the target period for which it will be tried to monitor diseases of a user. For example, when the estimation period is a period including a plurality of days including a point in time at which it will be tried to expect whether there is a disease, the (standardized) heart rate information for the estimation period may mean heart rate information for a partial period included in the plurality of days, but is not limited thereto.

For example, when the estimation period is a period including a plurality of days including the day before a point in time at which it will be tried to expect whether there is a disease, the (standardized) heart rate information for the estimation period may mean heart rate information for a partial period included in the plurality of days, but is not limited thereto. The first disease monitoring model may be a machine learning model, a deep learning model, or a combination thereof. The first disease monitoring model may be, for example, an RNN-series model such as an RNN model, an LSTM model, or a GRU model, but is not limited thereto.

The first disease monitoring model can be trained through various methods such as supervised learning, unsupervised learning, semi-supervised learning, and reinforcement learning.

For example, the first disease monitoring model may be trained through supervised learning. In this case, the first disease monitoring model can be trained using training (standardized) heart rate information and reference disease monitoring information corresponding to the training (standardized) heart rate information. As a more detailed example, the first disease monitoring model can receive training (standardized) heart rate information, can output disease monitoring information, and can be trained on the basis of the difference between the output disease monitoring information and the reference disease monitoring information. In this case, the (standardized) heart rate information may correspond to the (standardized) heart rate information for the estimation period.

The disease monitoring server can train the first disease monitoring model. Alternatively, the disease monitoring server can be provided with a trained first disease monitoring model from the outside.

Alternatively, the disease monitoring server can generate disease monitoring information by inputting (standardized) heart rate information for an estimation period and (standardized) heart rate information for a reference period into the first disease monitoring model.

The reference period may be a period for which the thyroid function is determined as being normal. The reference period may be a period including a point in time at which thyroid hormone values are determined as being normal. The reference period may be the period immediately before the day at which thyroid hormone values were measured through a blood test, a period including the measurement day, or a period immediately after the measurement day. The reference period may be the length of a predetermined period. The length of the reference period may be the same as the length of the estimation period. The method of calculating a representative heart rate for the reference period may be the same as the method of calculating a representative heart rate for the estimation period.

Further, the reference period may be, regardless of whether the thyroid function of a user is normal, a period immediately before a day at which thyroid hormone values of the user were measured through a blood test, a period including the measurement day, or a period immediately after the measurement day, that is, a period related to the day at which thyroid hormone values of the user were obtained, but is not limited thereto.

The estimation period may be after the reference period. The reference period may be before the estimation period. The estimation period may not overlap the reference period. The estimation period may overlap the reference period.

The first disease monitoring model may be trained similarly to the above description. However, the first disease monitoring model may be trained further using training (standardized) heart rate information corresponding to a reference period. For example, the first disease monitoring model can receive training (standardized) heart rate information and training (standardized) heart rate information corresponding to a reference period, can output disease monitoring information, and can be trained on the basis of the difference between the output disease monitoring information and the reference disease monitoring information.

Alternatively, the disease monitoring server can generate disease monitoring information by inputting the ratio between (standardized) heart rate information for an estimation period and (standardized) heart rate information for a reference period (hereafter, "heart rate information ratio") into the first disease monitoring model.

The first disease monitoring model may be trained similarly to the above description. However, in this case, the first disease monitoring model may be trained using a training heart rate information ratio instead of the training (standardized) heart rate information.

Alternatively, the disease monitoring server can generate disease monitoring information by inputting the difference between (standardized) heart rate information for an estimation period and (standardized) heart rate information for a reference period (hereafter, "heart rate information difference") into the first disease monitoring model.

The first disease monitoring model may be trained similarly to the above description. However, in this case, the first disease monitoring model may be trained using a training heart rate information difference instead of the training (standardized) heart rate information.

Further, the disease monitoring server can generate disease monitoring information using at least one of (standardized) heart rate information for an estimation period and (standardized) heart rate information for a reference period, and the first disease monitoring model.

Referring to FIG. 8B, a disease monitoring server according to another embodiment can generate one or more representative heart rates from (standardized) heart rate information and can generate disease monitoring information by inputting the one or more representative heart rates into a second disease monitoring model.

The representative heart rate may be a representative value that is calculated on the basis of (standardized) heart rate information for a certain period. For example, the representative heart rate may be an average value of (standardized) heart rate information in a section that satisfies a predetermined reference in a certain period. As another example, the representative heart rate may be a standard deviation value of (standardized) heart rate information in a section that satisfies a predetermined reference in a certain period. As another example, the representative heart rate may be a median of (standardized) heart rate information in a section that satisfies a predetermined reference in a certain period.

According to an embodiment, an algorithm for calculating a representative heart rate may be stored in a disease monitoring server.

A disease monitoring server can obtain a representative heart rate for an estimation period. For example, the controller of a disease monitoring server can obtain a representative heart rate for an estimation period by calculating a representative heart rate on the basis of (standardized) heart rate information for the estimation period stored in the storage. As another example, the controller of a disease monitoring server can obtain a representative heart rate for an estimation period by receiving data about (standardized) heart rate information for the estimation period through the communication unit and calculating a representative heart rate on the basis of the (standardized) heart rate information for the estimation period. As other example, the controller of a disease monitoring server can obtain a representative heart rate for an estimation period by receiving a representative heart rate for the estimation period through the communication unit.

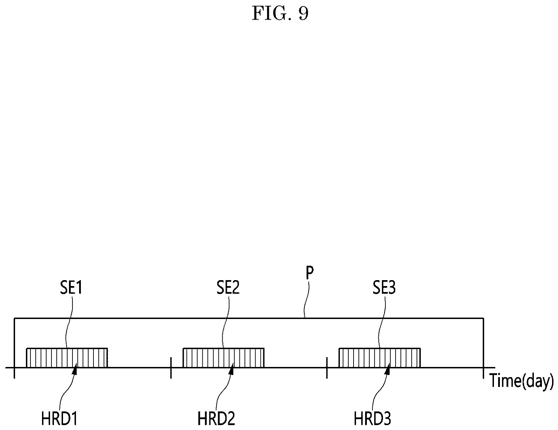

FIG. 9 is a view showing obtaining a representative heart rate according to an embodiment.

A disease monitoring server can extract at least one section that satisfies a predetermined reference in an estimation period P. For example, the disease monitoring server can extract a section corresponding to a time stored in a "sleep time" in the estimation period P. As a detailed example, when a sleep time is stored as a time from the 12 a.m. to 6 a.m., the disease monitoring server can extract sections corresponding to the time from 12 a.m. to 6 a.m. in the estimation period P. In this case, when the estimation period P is 3 days, up to 3 sections can be extracted.

Referring to FIG. 9, three sections discriminated as a first section SE1, a second section SE2, and a third section SE3 can be extracted as sections that satisfy a predetermined reference in the estimation period P of 3 days.

The disease monitoring server can calculate a representative value of (standardized) heart rate information included in an extracted section. The disease monitoring server can determine a representative value of (standardized) heart rate information included in an extracted section as a representative heart rate. The disease monitoring server can determine a representative value of (standardized) heart rate information included in a section extracted from a specific period as a representative heart rate for the specific period.

For example, the disease monitoring server can determine the average value of (standardized) heart rate information corresponding to the first section SE1, the second section SE2, and the third section SE3 as a representative heart rate. As another example, the disease monitoring server can determine the standard deviation value of (standardized) heart rate information corresponding to the first section SE1, the second section SE2, and the third section SE3 as a representative heart rate. As another example, the disease monitoring server can determine, as a representative heart rate, the average value of the average value of (standardized) heart rate information HRD1 corresponding to the first section, the average value of (standardized) heart rate information HRD2 corresponding to the second section, and the average value of (standardized) heart rate information HRD3 corresponding to the third section. As another example, the disease monitoring server can determine, as a representative heart rate, the standard deviation value of the average value of (standardized) heart rate information HRD1 corresponding to the first section, the average value of (standardized) heart rate information HRD2 corresponding to the second section, and the average value of (standardized) heart rate information HRD3 corresponding to the third section. As another example, the disease monitoring server can determine, as a representative heart rate, the average value of the median of (standardized) heart rate information HRD1 corresponding to the first section, the median of (standardized) heart rate information HRD2 corresponding to the second section, and the median of (standardized) heart rate information HRD3 corresponding to the third section.

The disease monitoring server can extract at least one section that satisfies a predetermined reference in the estimation period P. As another example, the disease monitoring server can extract a "section in which user's movement is little" in the estimation period P. As a detailed example, the disease monitoring server can extract a section that is determined as a rest section of a user in the estimation period P. The rest section may be a section that is sorted as a sleep section by at least one sensor, a section in which user's movement is 0 by a motion sensor, etc., or a section that is sorted as a rest section in an existing product.

In the specification, a sleep section and/or a section in which user's movement is little may be referred to as a rest section in combination. In the specification, heart rates pertaining to a sleep section and/or a section in which user's movement is little may be referred to as a rest section heart rate in combination.

The disease monitoring server can generate disease monitoring information by inputting a representative heart rate for an estimation period into the second disease monitoring model.

A second disease monitoring model may be a machine learning model, a deep learning model, or a combination thereof. The second disease monitoring model may be, for example, an RNN-series model such as an RNN model, an LSTM model, or a GRU model, but is not limited thereto.

The second disease monitoring model can be trained through various methods such as supervised learning, unsupervised learning, semi-supervised learning, and reinforcement learning.

For example, the second disease monitoring model may be trained through supervised learning. In this case, the second disease monitoring model can be trained using a training heart rate and reference disease monitoring information corresponding to the training heart rate. As a more detailed example, the second disease monitoring model can receive a training heart rate, can output disease monitoring information, and can be trained on the basis of the difference between the output disease monitoring information and the reference disease monitoring information. In this case, the training heart rate may correspond to the representative heart rate for the estimation period.

The disease monitoring server can train the second disease monitoring model. Alternatively, the disease monitoring server can be provided with a trained second disease monitoring model from the outside.

Alternatively, the disease monitoring server can generate disease monitoring information by inputting a representative heart rate for an estimation period and a representative heart rate for a reference period into the second disease monitoring model.

The disease monitoring server can obtain a representative heart rate for a reference period. For example, the controller of the disease monitoring server can obtain a representative heart rate for a reference period by loading a representative heart rate for a pre-calculated reference period stored in the storage. As another example, the controller of the disease monitoring server can obtain a representative heart rate for a reference period by calculating a representative heart rate on the basis of heart rate information for a reference period or standardized heart rate information stored in the storage. As another example, the controller of the disease monitoring server can obtain a representative heart rate for a reference period by receiving data about heart rate information or standardized heart rate information for the reference period through the communication unit and calculating a representative heart rate on the basis of the heart rate information for the reference period or the standardized heart rate information. As other example, the controller of the disease monitoring server can obtain a representative heart rate for a reference period by receiving a representative heart rate for the estimation period through the communication unit.

The second disease monitoring model may be trained similarly to the above description. However, the second disease monitoring model may be trained further using a training heart rate corresponding to a reference period. For example, the second disease monitoring model can receive a training heart rate and a training heart rate corresponding to a reference period, can output output disease monitoring information, and can be trained on the basis of the difference between the output disease monitoring information and the reference disease monitoring information.

Alternatively, the disease monitoring server can generate disease monitoring information by inputting the ratio between a representative heart rate for an estimation period and a representative heart rate for a reference period (hereafter, "representative heart rate ratio) into the second disease monitoring model.

The second disease monitoring model may be trained similarly to the above description. However, in this case, the second disease monitoring model may be trained using a training representative heart rate ratio instead of the training heart rate.

Alternatively, the disease monitoring server can generate disease monitoring information by inputting the difference between a representative heart rate for an estimation period and a representative heart rate for a reference period (hereafter, "representative heart rate difference") into the second disease monitoring model.

The second disease monitoring model may be trained similarly to the above description. However, in this case, the second disease monitoring model may be trained using a training representative heart rate difference instead of the training (standardized) heart rate information.

Further, the disease monitoring server can generate disease monitoring information using at least one of a representative heart rate for an estimation period and a representative heart rate for a reference period, and the second disease monitoring model.

Referring to FIG. 4 again, in step S460, the disease monitoring server can output disease monitoring information.

Outputting disease monitoring information by the disease monitoring server may mean transmitting disease monitoring information to an external device such as a user terminal. In this case, the external device receiving the disease monitoring information can display the disease monitoring information through a display thereof.

Outputting disease monitoring information by the disease monitoring server may mean displaying the disease monitoring information through a display included in the disease monitoring server.

Hereafter, as a modified example of the heart rate information standardization step, the case in which heart rate information has a missing value is described.

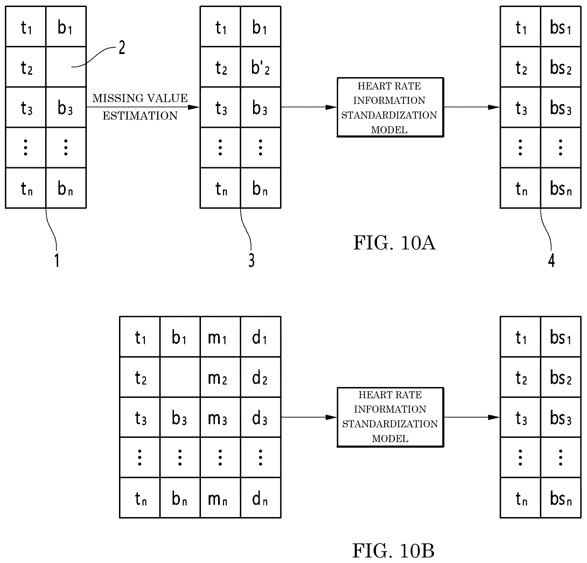

FIGS. 10A and 10B are views showing generating standardized heart rate information using heart rate information having a missing value according to an embodiment.

Referring to FIG. 10A, when heart rate information 1 has a missing value 2, a disease monitoring server according to an embodiment generates heart rate information 3 with a missing value estimated through a missing value estimation algorithm and inputs the heart rate information 3 with an estimated missing value into a heart rate information standardization model, thereby being able to generate standardized heart rate information 4.

Interpolation may be used as an example of the missing value estimation algorithm. For example, the disease monitoring server can estimate a missing value using a heart rate around the point in time at which a missing value exists in heart rate information. As a more detailed example, the disease monitoring server can determine a heart rate before the point in time at which a missing value exists as a heart rate at the point in time at which the missing value exists (e.g., when a heart rate at 3:01 is a missing value, the heart rate at 3:00 is determined as the heart rate at 3:01). As a more detailed embodiment, the disease monitoring server can determine a heart rate after the point in time at which a missing value exists as a heart rage at the point in time at which the missing value exists (e.g., when a heart rate at 3:01 is a missing value, the heart rate at 3:02 is determined as the heart rate at 3:01). As a more detailed example, the disease monitoring server can determine the average value of heart rates before and after the point in time at which a missing value exists as a heart rate at the point in time at which the missing value exists (e.g., when a heart rate at 3:01 is a missing value, the average value of the heart rate at 3:00 and the heart rate at 3:02 is determined as the heart rate at 3:01).

The missing value estimation algorithm is not limited to the way described above and various value estimation algorithms may be used. For example, the missing value estimation algorithm may be implemented into an autoregressive model, an RNN-series model such as a deep learning model based on gated recurrent unit (GRU-D) or bidirectional recurrent imputation for time series (BRITS), a GAN-series model such as non-autoregressive multiresolution sequence imputation (NAOMI).

Referring to FIG. 10B, a disease monitoring server according to another embodiment can generate standardized heart rate information using a heart rate information standardization model in which missing value processing and heart rate information standardization are integrated, without a specific missing value estimation step shown in FIG. 10A. The integrated model, for example, may be an RNN-series model such as a deep learning model based on gated recurrent unit (GRU-D) or bidirectional recurrent imputation for time series (BRITS), or a GAN-series model such as non-autoregressive multiresolution sequence imputation (NAOMI), but is not limited thereto. When GRU-D is used, as shown in FIG. 10B, input data that are input into the model may include maskings m1 to mn corresponding to whether a missing value exists, and intervals d1 to dn corresponding to the interval from a point in time before a missing value exists.

The case in which whether to generate standardized heart rate information depends on whether type information corresponds to a reference type was described above, but, in some embodiments, a disease monitoring server may generate standardized heart rate information and generate disease monitoring information using the standardized heart rate information, regardless of type information.

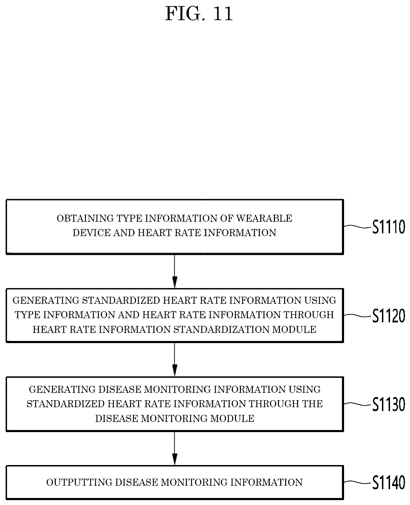

FIG. 11 is a flowchart showing a disease monitoring method according to another embodiment.

In step S1110, a disease monitoring server can obtain type information of a wearable device and heart rate information. The matter of step S410 described above can be similarly applied to this step, so repeated description is omitted.

In step S1120, the disease monitoring server can generate standardized heart rate information using type information and heart rate information through a heart rate information standardization module. The matter in step S430 described above can be similarly applied to this step, so configuration repeated to the description of step S430 is briefly or not described hereafter.

According to an embodiment, the heart rate information standardization module may be implemented using a heart rate information standardization model.

The disease monitoring server can generate standardized heart rate information using heart rate information, type information, and a heart rate information standardization model. For example, the disease monitoring server can generate standardized heart rate information by inputting heart rate information and type information into the heart rate information standardization model. As another example, the disease monitoring server can generate standardized heart rate information by inputting information generated from heart rate information and type information into a heart rate information standardization model.

The heart rate information may be heart rate information obtained by a wearable device and the standardized heart rate information may be heart rate information obtained from a heart rate information measurement device other than the wearable device or heart rate information expected as being obtained from a heart rate information measurement device other than the wearable device.

The heart rate information measurement device may be a device known as measuring heart rate information more accurately than wearable devices or a device specialized to measure heart rate information. The heart rate information measurement device may be, for example, a heart rate monitor, but is not limited thereto.

A heart rate information standardization model may be a machine learning model, a deep learning model, or a combination thereof. The heart rate information standardization model may be, for example, an RNN-series model such as an RNN model, an LSTM model, or a GRU model, but is not limited thereto.

The heart rate information standardization model can be trained through various methods such as supervised learning, unsupervised learning, semi-supervised learning, and reinforcement learning.

The matter described above with reference to FIG. 4 can be similarly applied to training the heart rate information standardization model. However, the heart rate information may be heart rate information obtained by a wearable device and reference heart rate information may be heart rate information obtained from a heart rate information measurement device other than the wearable device or heart rate information expected as being obtained from a heart rate information measurement device other than the wearable device.

According to another embodiment, the heart rate information standardization module may be implemented using a plurality of type models. For example, the heart rate information standardization module may be implemented using at least a first type model and a second type model.

The disease monitoring server can generate standardized heart rate information using different type models in accordance with the types of wearable devices.

The disease monitoring server can generate standardized heart rate information using heart rate information, type information, and a first type model when the type information of a wearable device corresponds to a first type. For example, the disease monitoring server can generate standardized heart rate information by inputting heart rate information and type information into a first type model when the type information of a wearable device corresponds to a first type. As another example, the disease monitoring server can generate standardized heart rate information by inputting information generated from heart rate information and type information into the first type model when the type information of a wearable device corresponds to the first type.

The disease monitoring server can generate standardized heart rate information using heart rate information, type information, and a second type model when the type information of a wearable device corresponds to a second type. For example, the disease monitoring server can generate standardized heart rate information by inputting heart rate information and type information into the second type model when the type information of a wearable device corresponds to the second type. As another example, the disease monitoring server can generate standardized heart rate information by inputting information generated from heart rate information and type information into the second type model when the type information of a wearable device corresponds to the second type.

The above description of implementing a heart rate information standardization module using a heart rate information standardization model in accordance with an embodiment can be similarly applied to the heart rate information and the standardized heart rate information, so repeated description is omitted.

A type model may be a machine learning model, a deep learning model, or a combination thereof. The first type model may be, for example, an RNN-series model such as an RNN model, an LSTM model, or a GRU model, but is not limited thereto.

The type model can be trained through various methods such as supervised learning, unsupervised learning, semi-supervised learning, and reinforcement learning.

The matter described above with reference to FIG. 4 can be similarly applied to training the type model. However, reference heart rate information may be heart rate information obtained from a heart rate information measurement device other than a wearable device or heart rate information expected as being obtained from a heart rate information measurement device other than a wearable device.

In step S1130, the disease monitoring server can generate disease monitoring information using standardized heart rate information through the disease monitoring module. The above description of generating disease monitoring information in steps S440 and S450 can be similarly applied to this step, so repeated description is omitted.

In step S1140, the disease monitoring server can output disease monitoring information. The matter of step S460 described above can be similarly applied to this step, so repeated description is omitted.

Some embodiments of performing a disease monitoring method in a disease monitoring system are described hereafter.

The above description can be applied to steps that components perform in each example, so repeated description is omitted.

A disease monitoring system may perform a disease monitoring method in ways different from examples to be described below.

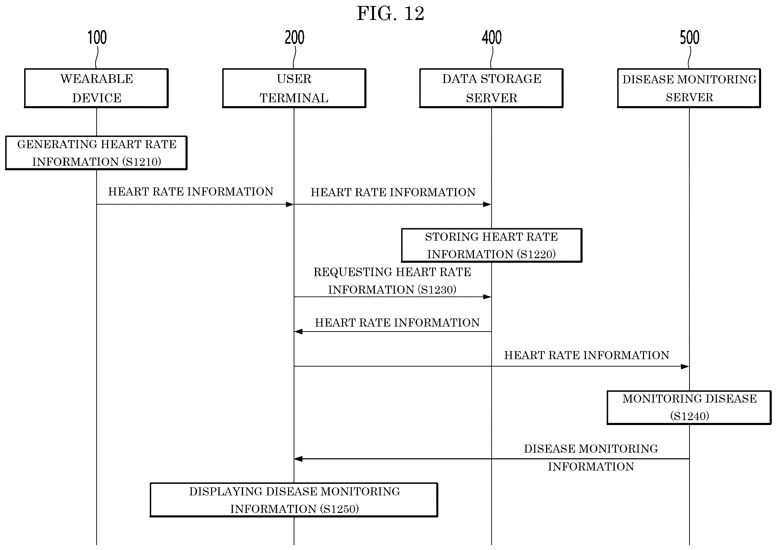

FIG. 12 is a flowchart showing a first example of performing a disease monitoring method in a disease monitoring system according to an embodiment. Referring to FIG. 12, a data storage server can store heart rate information obtained by a wearable device and a disease monitoring server can receive stored heart rate information through a user terminal and perform a disease monitoring method.

According to the first example, the disease monitoring system may include a wearable device, a user terminal, a data storage server, and a disease monitoring server.

The wearable device can sense heart rate information of a user. The wearable device can generate heart rate information (S1210).

The wearable device can transmit the heart rate information to the user terminal.

The user terminal can transmit the heart rate information received from the wearable device to the data storage server.

The data storage server can store the heart rate information (S1220).

The user terminal can request heart rate information from the data storage server (S1230).

Selectively, the data storage server can identify whether the user terminal has been authorized to access heart rate information.

In accordance the request from the user terminal, the data storage server can transmit the stored heart rate information to the user terminal. Selectively, the data storage server can transmit the stored heart rate information to a user terminal that has been authorized to access heart rate information.

The user terminal can transmit the heart rate information to the disease monitoring server.

The disease monitoring server can perform the disease monitoring method described above (S1240). The disease monitoring server can generate disease monitoring information.

The disease monitoring server can transmit the generated disease monitoring information to the user terminal.

The user terminal can display the received disease monitoring information on a display (1250).

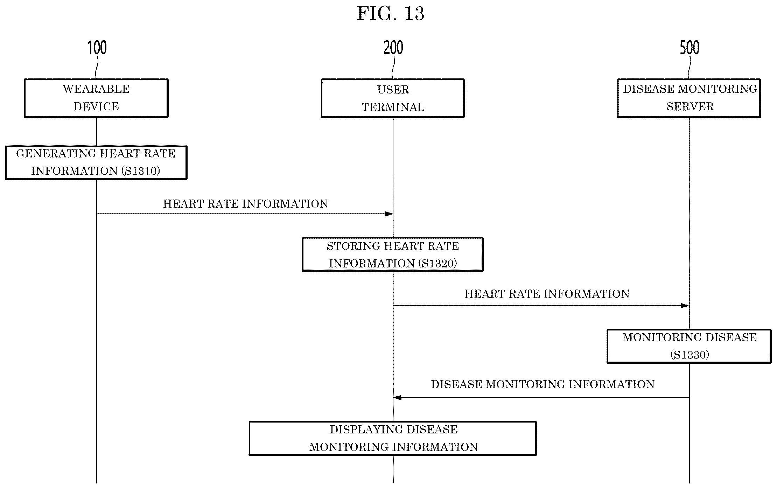

FIG. 13 is a flowchart showing a second example of performing a disease monitoring method in a disease monitoring system according to an embodiment. Referring to FIG. 13, a user terminal can store heart rate information obtained by a wearable device and a disease monitoring server can receive stored heart rate information from the user terminal and perform a disease monitoring method.

According to the second example, the disease monitoring system may include a wearable device, a user terminal, and a disease monitoring server.

The wearable device can sense heart rate information of a user. The wearable device can generate heart rate information (S1310).

The wearable device can transmit heart rate information to the user terminal.

The user terminal can store the heart rate information (S1320).

The user terminal can transmit the heart rate information to the disease monitoring server.

The disease monitoring server can perform the disease monitoring method described above (S1330). The disease monitoring server can generate disease monitoring information.

The disease monitoring server can transmit the generated disease monitoring information to the user terminal.

The user terminal can display the received disease monitoring information on a display (1340).

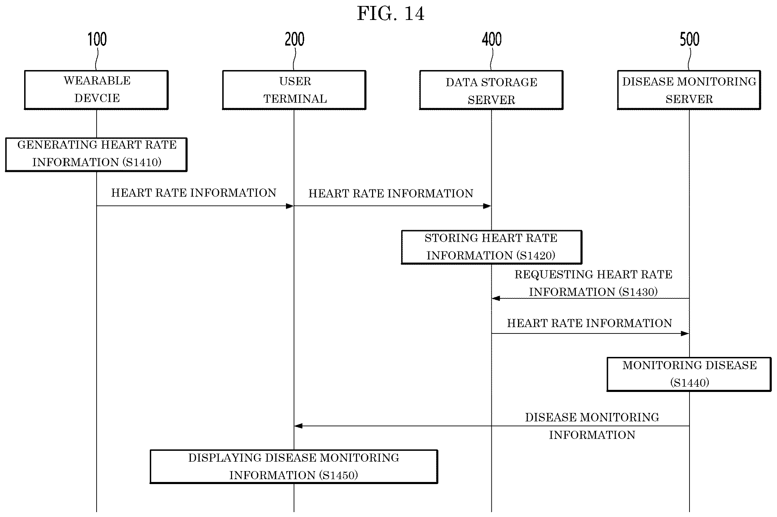

FIG. 14 is a flowchart showing a third example of performing a disease monitoring method in a disease monitoring system according to an embodiment. Referring to FIG. 14, a data storage server can store heart rate information obtained by a wearable device and a disease monitoring server can receive stored heart rate information from the data storage server and perform a disease monitoring method.

According to the third example, the disease monitoring system may include a wearable device, a user terminal, a data storage server, and a disease monitoring server.

The wearable device can sense heart rate information of a user. The wearable device can generate heart rate information (S1410).

The wearable device can transmit heart rate information to the user terminal.

The user terminal can transmit the heart rate information received from the wearable device to the data storage server.

The data storage server can store the heart rate information (S1420).

The disease monitoring server can request heart rate information from the data storage server (S1430).

Selectively, the data storage server can identify whether the data storage server has been authorized to access heart rate information.

In accordance with the request from the disease monitoring server, the data storage server can transmit the stored heart rate information to the disease monitoring server. Selectively, the data storage server can transmit the stored heart rate information to a disease monitoring server that has been authorized to access heart rate information.

The disease monitoring server can perform the disease monitoring method described above (S1440). The disease monitoring server can generate disease monitoring information.

The disease monitoring server can transmit the generated disease monitoring information to the user terminal.

The user terminal can display the received disease monitoring information on a display (1450).

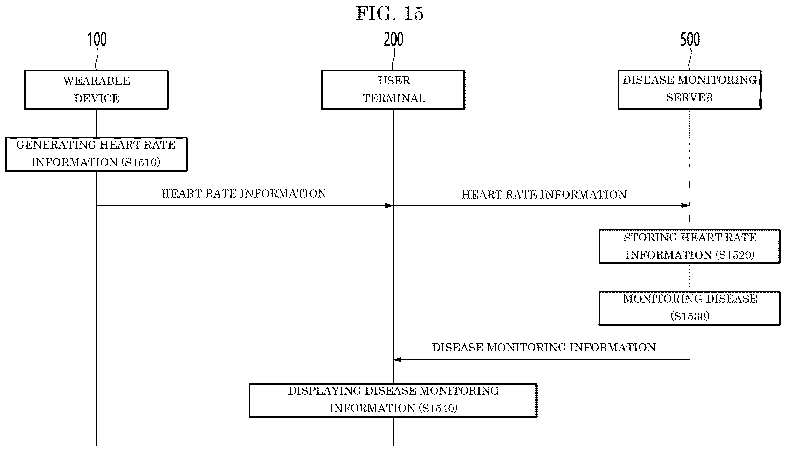

FIG. 15 is a flowchart showing a fourth example of performing a disease monitoring method in a disease monitoring system according to an embodiment. Referring to FIG. 15, a disease monitoring server can store heart rate information obtained by a wearable device and can perform a disease monitoring method using the heart rate information.

According to the fourth example, the disease monitoring system may include a wearable device, a user terminal, and a disease monitoring server.

The wearable device can sense heart rate information of a user. The wearable device can generate heart rate information (S1510).

The wearable device can transmit heart rate information to the user terminal.

The user terminal can transmit the heart rate information to the disease monitoring server.

The disease monitoring server can store the heart rate information (S1520).

The disease monitoring server can perform the disease monitoring method described above (S1530). The disease monitoring server can generate disease monitoring information.

The disease monitoring server can transmit the generated disease monitoring information to the user terminal.

The user terminal can display the received disease monitoring information on a display (1540).

The disease monitoring method may further include a user registration step.

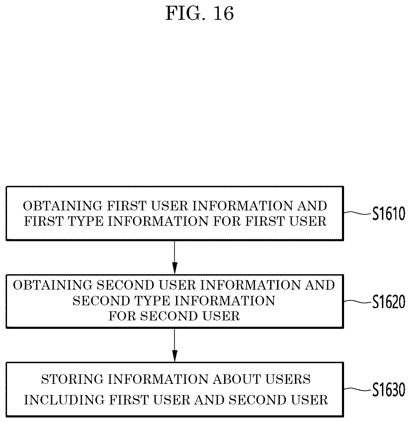

FIG. 16 is a flowchart showing a user registration step according to an embodiment.

In step S1610, a disease monitoring server can obtain first user information and first type information about a first user. For example, the disease monitoring server can obtain the first user information and the first type information from a user terminal. In this case, the first type information may be received from a wearable device. Alternatively, the first type information may be generated by the user terminal. As another example, the disease monitoring server can receive the first user information and the first type information from the wearable device.

In step S1620, the disease monitoring server can obtain second user information and second type information about a second user. The matter of step S1610 described above can be similarly applied to this step, so repeated description is omitted.

In step S1630, the disease monitoring server can store information about users including the first user and the second user. For example, the disease monitoring server can store the information about users into a user information database as a database.

Meanwhile, monitoring diseases on the basis of heart rate information was described above, but the above description can be similarly applied to monitoring diseases on the basis of biological information other than heart rate information.

(Example 1) It can be applied to monitoring diseases such as thyroid dysfunction on the basis of skin conductance. In this case, the skin conductance can be measured through a skin conductance measurement sensor such as an Electrodermal Activity (EDA) sensor. The sensor may be a sensor mounted on a wearable device.

(Example 2) It can be applied to diagnose motility of bowels or monitoring diseases related to bowels on the basis of a bowel sound. In this case, the bowel sound can be measured through a bowel sound measurement sensor such as a sound sensor. The sensor may be a sensor mounted on a wearable device.

(Example 3) It can be applied to monitoring diseases such as apnea on the basis of a blood oxygen level. In this case, the blood oxygen level can be measured through a sensor such as a pulse oximeter. The sensor may be a sensor mounted on a wearable device.

(Example 4) It can be applied to monitoring diseases on the basis of a motion sensor. The motion sensor may include an acceleration sensor, a gyro sensor, a step counter sensor, etc.

(Example 5) It can be applied to monitoring diseases such as hypertension or hypotension on the basis of blood pressure. In this case, the blood pressure can be measured by a blood pressure sensor. The blood pressure sensor may be a sensor mounted on a wearable device.

(Example 6) It can be applied to monitoring diseases such as diabetes on the basis of blood glucose. In this case, the blood glucose can be measured through a blood glucose sensor. The blood glucose sensor may be a sensor mounted on a wearable device.

(Example 7) It can be applied to monitoring diseases on the basis of body temperature. In this case, the body temperature can be measured through a temperature sensor such as an IR temperature sensor. The temperature sensor may be a sensor mounted on a wearable device.

(Example 8) It can be applied to monitoring diseases on the basis of a respiratory rate. In this case, the respiratory rate can be measured through a respiratory rate sensor. The respiratory rate sensor may be a sensor mounted on a wearable device.

(Example 9) It can be applied to monitoring diseases on the basis of skin moisture. In this case, the skin moisture can be measured through a skin moisture sensor. The skin moisture sensor may be a sensor mounted on a wearable device.

(Example 10) It can be applied to monitoring diseases on the basis of stress or the concentration of a stress hormone. In this case, the stress can be measured through a stress sensor. Further, the concentration of a stress hormone can be measured through a stress hormone concentration sensor. The stress sensor or the stress hormone concentration sensor may be a sensor mounted on a wearable device.

A method according to an embodiment may be performed by a processing logic including hardware, firmware, software, or a combination thereof. A method according to an embodiment may be performed by a processor that executes codes stored in a non-transitory computer-readable medium. The non-transitory computer-readable medium include magnetic media such as hard disks, floppy disks, and magnetic media such as a magnetic tape, optical media such as CD-ROMs and DVDs, magneto-optical media such as floptical disks, and hardware devices specifically configured to store and execute program commands and instructions, such as ROM, RAM, and flash memory.

Although the present disclosure was described above on the basis of embodiments, the present disclosure is not limited thereto and it is apparent to those skilled in the art that the present disclosure may be changed and modified in various ways within the spirit and scope of the present disclosure, so such it should be noted that such changes and modifications are included in the accompanying claims.

Hereafter, experimental examples of generating standardized heart rate information from heart rate information in accordance with type information of wearable devices are disclosed. The following experimental examples were not intended to limit the scope of the present disclosure in any way.

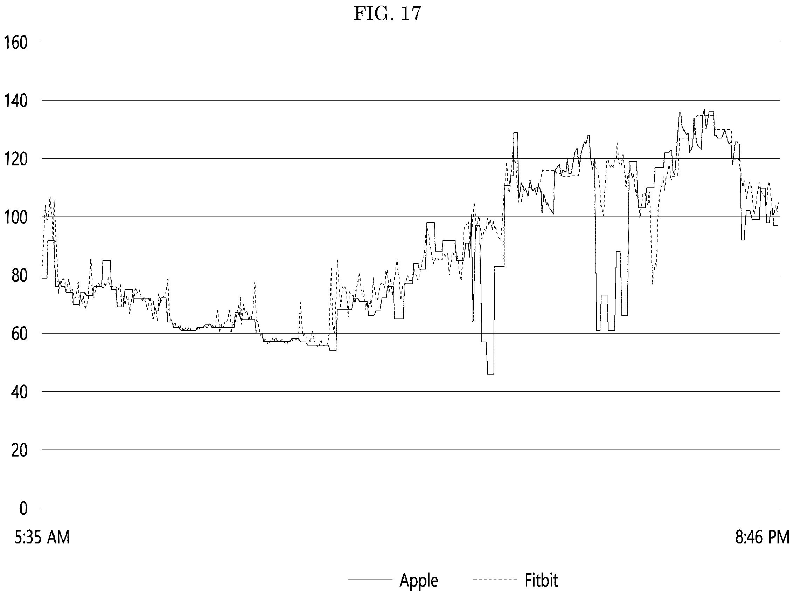

FIG. 17 is a graph showing the result of measuring heart rate information of a specific user through Apple Watch and Fitbit, that is, a graph showing the result of measuring heart rate information with a specific user wearing Apple Watch and Fitbit on the same wrist. The graph shows heart rate information measured from 5:35 a.m. to 8:46 p.m. on October 19, 2021, in which the x axis represents time and the y axis represents a heart rate.

The Fitbit measures a heart rate in the unit of 1 minute but the Apple Watch non-periodically measures a heart rate with an interval over 1 minute, so a heart rate at a point in time around a missing value was added for the missing value in the heart rate information measured by the Apple Watch to make a heart rate in the unit of 1 minute.

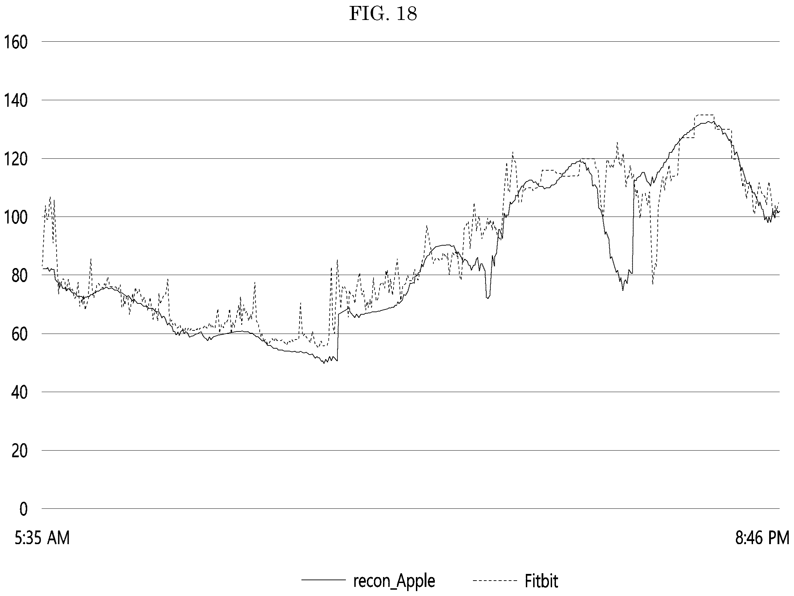

FIG. 18 is a graph showing standardizing heart rate information in accordance with the type information of a wearable device when Fitbit is a reference type. Since Fitbit is a reference type and the heart rate information measured by the Fitbit shown in FIG. 17 has been already standardized, so a heart rate standardization step was not performed. However, since Apple Watch is not a reference type, standardized heart rate information was measured from the heart rate information measured by the Apple Watch shown in FIG. 17 through a heart rate information standardization step. In FIG. 18, "recon_Apple" means standardized heart rate information generated from heart rate information measured by Apple Watch.

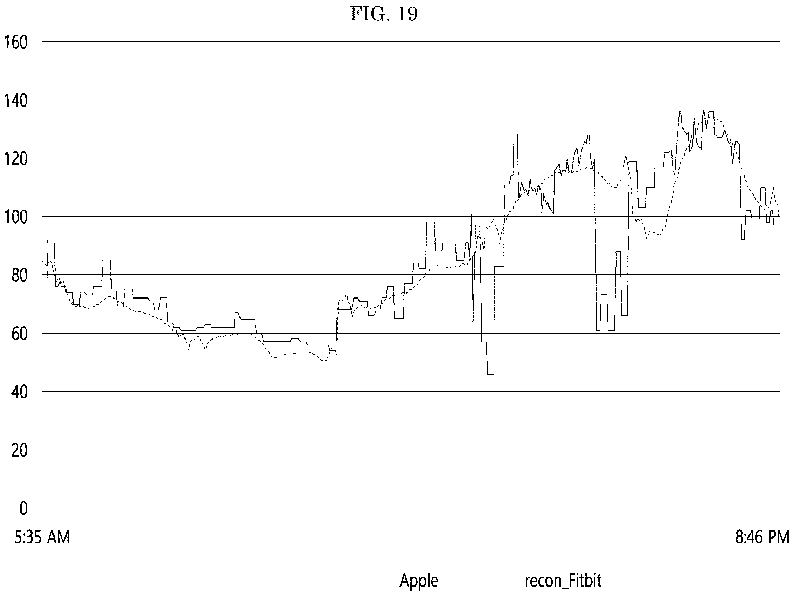

FIG. 19 is a graph showing standardized heart rate information in accordance with the type information of a wearable device when Apple Watch is a reference type. Since Apple Watch is a reference type and the heart rate information measured by the Apple Watch shown in FIG. 17 has been already standardized, so a heart rate standardization step was not performed. However, since Fitbit is not a reference type, standardized heart rate information was measured from the heart rate information measured by the Fitbit shown in FIG. 17 through a heart rate information standardization step. In FIG. 19, "recon_Fitbit" means standardized heart rate information generated from heart rate information measured by Fitbit.

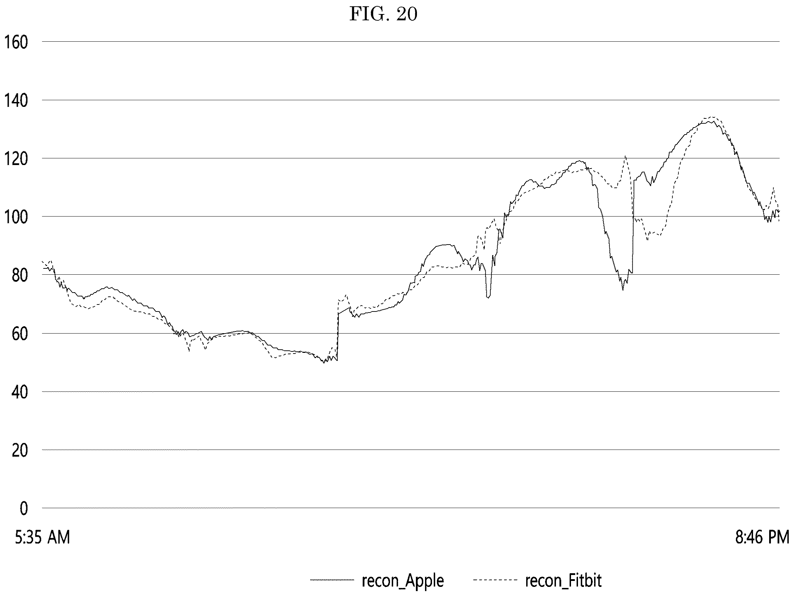

FIG. 20 is a graph showing the standardized heart rate information of both FIG. 18 and FIG. 19. Comparing FIG. 17 and FIG. 20, it was found that similarity of standardized heart rate information increased before heart rate was standardized. In particular, it was found that similarity was high for a sleep time such as the midnight hours or a time for which user's activity is little rather than daytime for which user actively moves.

Further, the average value of the differences of the heart rate information obtained by the Apple Watch and the Fitbit shown in FIG. 17 is 7.452, the standard deviation value is 11.559, the distribution value is 133.619, and the number of differences in heart rate over 10 is 105.

Further, the average value of the differences of the heart rate information obtained by "recon_Apple" and the Fitbit shown in FIG. 18 is 6.637, the standard deviation value is 7.935, the distribution value is 62.959, and the number of differences in heart rate over 10 is 91.

Further, the average value of the differences of the heart rate information obtained by the Apple Watch shown in FIG. 19 and "recon_Fitbit" is 8.206, the standard deviation value is 10.744, the distribution value is 115.428, and the number of differences in heart rate over 10 is 95.

Further, the average value of the differences of "recon_Apple" and "recon_Fitbit" shown in FIG. 20 is 4.924, the standard deviation value is 7.073, the distribution value is 50.025, and the number of differences in heart rate over 10 is 65.

Therefore, referring to FIGS. 17 to 20, it is possible to see that similarity of standardized heart rate information increased more than before heart rate was standardized, and particularly, the standard deviation value of the differences in standardized heart rate information decreased under 10, so the possibility that different types of heart rate information can be applied (learned or used) for the same disease monitoring model could increase.

A method and model for standardizing heart rate information, and a disease monitoring method using standardized heart rate information according to the present disclosure were described above.

According to the above description of the present disclosure, since it is possible to standardize heart rate information obtained from different types of devices, the difference of different types of heart rate information obtained from different types of devices is decreased, whereby it is possible to reduce difference in accuracy of a disease monitoring system that uses different types of devices.

Further, according to the above description of the present disclosure, since it is possible to standardize heart rate information obtained from different types of devices, it is possible to use different types of heart rate information obtained from different types of devices as training data for one disease monitoring model, so it is possible to more easily obtain training data for a disease monitoring model and secure more training data.

For example, when the heart rate standardization model according to the above description of the present disclosure is not used, the accuracy of a model may decrease due to the differences of different types of heart rate information obtained from different types of devices when the different types of heart rate information are used as training data for one disease monitoring model, however, when the heart rate standardization model according to the above description of the present disclosure is used, the differences of different types of heart rate information obtained from different types of devices are decreased, so the accuracy of a model can be increased when the different types of heart rate information are used as training data for one disease monitoring model.

Further, according to the above description of the present disclosure, since it is possible to standardize heart rate information obtained from different types of devices, the necessity of generating a plurality of disease monitoring models for each of different types of heart rate information obtained from different types of devices, respectively, can be reduced.

For example, when the heart rate information standardization model according to the above description of the present disclosure is not used, it is required to generate a disease monitoring model for each of different types of heart rate information obtained from different types of devices, respectively, due to the differences of the different types of heart rate information obtained from the different types of devices. However, when the heart rate standardization model according to the above description of the present disclosure is used, the differences of different types of heart rate information obtained from different types of devices are decreased, so it is possible to use the different types of heart rate information as training data for one disease monitoring model, whereby the necessity of generating a plurality of disease monitoring models can be reduced.

Further, the disease monitoring model according to the above description of the present disclosure may need to be approved through clinical tests, depending on the countries that use the disease monitoring model of necessity thereof.

In this case, when the heart rate information standardization model according to the above description of the present disclosure is used, it may be possible to be easily approved through clinical tests for a plurality of devices.

Further, in this case, when the heart rate information standardization model according to the above description of the present disclosure is used, it may be possible to be easily approved through clinical tests for following devices.

For example, assuming that a disease monitoring model using heart rate information obtained from a first-version wearable device by a specific manufacturer has been approved through a clinical test and then a second-version wearable device by the same manufacturer has been released, when the heart rate standardization model according to the above description of the present disclosure is not used, it is required to generate a new disease monitoring model by collecting again training data from the second-version wearable device and plan a clinical test for the new disease monitoring model. However, when the heart rate standardization model according to the above description of the present disclosure is used, it is possible to generate standardized heart rate information related to heart rate information obtained from the first-version wearable device on the basis of heart rate information obtained from the second-version wearable device and use the generated standardized heart rate information, so it is possible to plan a clinical test without generating a new disease monitoring model.

Therefore, according to an embodiment, a method of distributing a disease monitoring model may be provided.

A method of distributing a disease monitoring model according to an embodiment may include: training a disease monitoring model using heart rate information obtained from a first type device; obtaining a first disease monitoring result through the trained disease monitoring model on the basis of first type heart rate information obtained from the first type device on the body of a first user; obtaining a first diagnosis result by at least one doctor for the first user; obtaining a first comparison result for the first disease monitoring result and the first diagnosis result by the at least one doctor; obtaining a result of approving a disease monitoring model for the first type device on the basis of the first comparison result; distributing a disease monitoring model that can be used for the first type device; generating standardized heart rate information on the basis of second type heart rate information from a second type device on the body of a second user-the second type device being a device released after a point in time at which the disease monitoring model is trained; obtaining a second disease monitoring result through the trained disease monitoring model on the basis of the generated standardized heart rate information; obtaining a second diagnosis result by at least one doctor for the second user; obtaining a second comparison result for the second disease monitoring result and the second diagnosis result by the at least one doctor; obtaining a result of approving a disease monitoring model for the second type device on the basis of the second comparison result; and distributing a disease monitoring model that can be used for the second type device.

In this case, training data used for training a trained disease monitoring model may not include second type heart rate information obtained from the second type device. Further, according to an embodiment, a clinical test method for a disease monitoring model may be provided.

A clinical test method for a disease monitoring model according to an embodiment may include: preparing a disease monitoring system approved already through a clinical test; preparing a heart rate standardization model trained using training data obtained using another heart rate information obtaining device different from a heart rate information obtaining device used when training the disease monitoring system; inputting an output value of the heart rate standardization model into the disease monitoring system and preparing a clinical test target product; preparing clinical test data-the clinical test data including heart rate information obtained by the another heart rate information obtaining device and an evaluation value by a doctor for a heart rate obtaining target (that is, a patient); and calculating accuracy in estimating the evaluation value by the doctor by inputting the clinical test data into the clinical test target product.

As described above, related matters were described in a best mode of the present disclosure.

**Claims**

1. A method of monitoring diseases of a user on the basis of heart rate information obtained by a wearable device, the method comprising:

   obtaining type information of the wearable device and the heart rate information;
   generating disease monitoring information using the heart rate information through a disease monitoring module when the type information corresponds to a reference type;
generating standardized heart rate information using the heart rate information through a heart rate information standardization module and generating the disease monitoring information using the standardized heart rate information through the disease monitoring module when the type information does not correspond to the reference type; and
outputting the disease monitoring information.

2. The method of claim 1, wherein the heart rate information standardization module is implemented using a heart rate information standardization model,

   the heart rate information standardization model is a model trained using training heart rate information and reference heart rate information corresponding to the training heart rate information,
   the training heart rate information is heart rate information obtained by a wearable device that is not the reference type for a first time, and
   the reference heart rate information is heart rate information obtained by a wearable device that is the reference type for the first time.

3. The method of claim 1, wherein the disease monitoring module is implemented using a first disease monitoring model,

   the generating of the disease monitoring information using the heart rate information includes generating the disease monitoring information by inputting the heart rate information into the first disease monitoring model, and
   the generating of the disease monitoring information using the standardized heart rate information includes generating the disease monitoring information by inputting the standardized heart rate information into the first disease monitoring model.

4. The method of claim 1, wherein the disease monitoring module is implemented using a second disease monitoring model,
   the generating of the disease monitoring information using the heart rate information includes:

   generating a first rest period heart rate from the heart rate information; and
   generating the disease monitoring information by inputting the first rest period heart rate into the second disease monitoring model, and
   the generating of the disease monitoring information using the standardized heart rate information includes:

> generating a second rest period heart rate from the standardized heart rate information; and
> generating the disease monitoring information by inputting the second rest period heart rate into the second disease monitoring model.

5. The method of claim 4, wherein the disease monitoring module is implemented further using a rest period heart rate generation model, and
the first rest period heart rate and the second rest period heart rate are generated by inputting the heart rate information and the standardized heart rate information into the rest period heart rate generation model, respectively.

6. The method of claim 1, wherein the heart rate information includes a plurality of heart rates corresponding to different points in time, and
the standardized heart rate information includes a plurality of standardized heart rates corresponding to different points in time.

7. The method of claim 6, further comprising generating one or more heart rate missing value from the plurality of heart rates, thereby generating missing value estimation heart rate information including the plurality of heart rates and the heart rate missing value, wherein the standardized heart rate information is generated through the heart rate information standardization module using the missing value estimation heart rate information.

8. The method of claim 1, wherein the heart rate information standardization module is implemented using a heart rate information standardization model, and
the standardized heart rate information is generated by inputting the heart rate information into the heart rate information standardization model.

9. The method of claim 1, wherein the heart rate information standardization module is implemented using a first type model and a second type model,

> the standardized heart rate information is generated using the heart rate information and the first type model when the type information corresponds to a first type that is not the reference type, and
> the standardized heart rate information is generated using the heart rate information and the second type model when the type information corresponds to a second type that is not the reference type.

10. The method of claim 1, wherein the heart rate information standardization module is implemented using a sleep model or a non-sleep model,

> the standardized heart rate information is generated using heart rate information obtained in sleep and the sleep model when the heart rate information is the heart rate information obtained in sleep, and
> the standardized heart rate information is generated using heart rate information obtained in non-sleep and the non-sleep model when the heart rate information is the heart rate information obtained in non-sleep.

11. A method of monitoring diseases of a user on the basis of heart rate information obtained by a wearable device, the method comprising:

> obtaining type information of the wearable device and the heart rate information;
> generating standardized heart rate information using the type information and the heart rate information through a heart rate information standardization module;
> generating disease monitoring information using the standardized heart rate information through a disease monitoring module; and
> outputting the disease monitoring information.

12. The method of claim 11, wherein the heart rate information standardization module is implemented using a heart rate information standardization model,

> the heart rate information standardization model is a model trained using training heart rate information and reference heart rate information corresponding to the training heart rate information,
> the training heart rate information is heart rate information obtained by a wearable device for a first time, and
> the reference heart rate information is heart rate information obtained by a heart rate information measurement device for the first time.

13. The method of claim 11, wherein the disease monitoring module is implemented using a first disease monitoring model, and
the generating of the disease monitoring information includes generating the disease monitoring information by inputting the standardized heart rate information into the first disease monitoring model.

14. The method of claim 11, wherein the disease monitoring module is implemented using a second disease monitoring model, and

the generating of the disease monitoring information includes:

generating a rest period heart rate from the standardized heart rate information; and generating the disease monitoring information by inputting the rest period heart rate into the second disease monitoring model.

15. The method of claim 14, wherein the disease monitoring module is implemented further using a rest period heart rate generation model, and

the rest period heart rate is generated by inputting the standardized heart rate information into the rest period heart rate generation model.

16. The method of claim 11, wherein the heart rate information includes a plurality of heart rates corresponding to different points in time, and

the standardized heart rate information includes a plurality of standardized heart rates corresponding to different points in time.

17. The method of claim 16, further comprising generating one or more heart rate missing value from the plurality of heart rates, thereby generating missing value estimation heart rate information including the plurality of heart rates and the heart rate missing value,

wherein the standardized heart rate information is generated through the heart rate information standardization model using the missing value estimation heart rate information.

18. The method of claim 11, wherein the heart rate information standardization module is implemented using a heart rate information standardization model, and

the generating of the standardized heart rate information includes generating the standardized heart rate information by inputting the type information and the heart rate information into the heart rate information standardization model.

19. The method of claim 11, wherein the heart rate information standardization module is implemented using a first type model and a second type model,

the standardized heart rate information is generated using the heart rate information and the first type model when the type information corresponds to a first type, and

the standardized heart rate information is generated using the heart rate information and the second type model when the type information corresponds to a second type.

20. A non-transitory computer-readable medium that stores one or more instructions, wherein when the one or more instructions are executed by one or more processors of a device/server, the one or more instructions make the device/server perform the method of any one of claims 1 to 19.

21. A server for monitoring diseases of a user on the basis of heart rate information obtained by a wearable device, the server comprising:

a communication unit;

a storage configured to store one or more instructions; and

a controller configured to execute the one or more instructions stored in the storage,

wherein the controller executes the one or more instructions, thereby

obtaining type information of the wearable device and the heart rate information;

generating disease monitoring information using the heart rate information through a disease monitoring module when the type information corresponds to a reference type;

generating standardized heart rate information using the heart rate information through a heart rate information standardization module and generating the disease monitoring information using the standardized heart rate information through the disease monitoring module when the type information does not correspond to the reference type; and

outputting the disease monitoring information.

FIG. 1

EP 4 497 375 A1

FIG. 2

FIG. 3

DISEASE MONITORING SERVER (500)

COMMUNICATION DEVICE (510)

CONTROLLER (530)

STORAGE (520)

MODULE DEVICE (540)

HEART RATE INFORMATION STANDARDIZATION MODULE (541)

DISEASE MONITORING MODULE (542)

# FIG. 4

```
┌─────────────────────────────────────────┐
│  OBTAINING TYPE INFORMATION OF WEARABLE  │──── S410
│     DEVICE AND HEART RATE INFORMATION    │
└─────────────────────────────────────────┘
                    │
                    ▼
        YES    ╱─────────────╲
  ◄────────────   REFERENCE    ────────────── S420
  │            ╲   TYPE ?    ╱
  │             ╲───────────╱
  │                  │ NO
  │                  ▼
  │      ┌─────────────────────────────────────────┐
  │      │ GENERATING STANDARDIZED HEART RATE       │
  │      │ INFORMATION USING HEART RATE INFORMATION │──── S430
  │      │ THROUGH HEART RATE INFORMATION           │
  │      │ STANDARDIZATION MODULE                   │
  │      └─────────────────────────────────────────┘
  │                  │
  ▼                  ▼
┌──────────────────────────┐  ┌──────────────────────────────────┐
│ GENERATING DISEASE       │  │ GENERATING DISEASE MONITORING    │
│ MONITORING INFORMATION   │  │ INFORMATION USING STANDARDIZED    │──── S440
│ USING HEART RATE         │  │ HEART RATE INFORMATION THROUGH    │
│ INFORMATION THROUGH      │  │ DISEASE MONITORING MODULE         │
│ DISEASE MONITORING       │  └──────────────────────────────────┘
│ MODULE                   │                 │
└──────────────────────────┘                 ▼
   S450            │      ┌──────────────────────────────────┐
                   └─────►│ OUTPUTTING DISEASE MONITORING     │──── S460
                          │ INFORMATION                       │
                          └──────────────────────────────────┘
```

28

| $t_1$ | $b_1$ |
|---|---|
| $t_2$ | $b_2$ |
| ⋮ | ⋮ |
| $t_n$ | $b_n$ |

HEART RATE INFORMATION
STANDARDIZATION MODEL

| $t_1$ | $bs_1$ |
|---|---|
| $t_2$ | $bs_2$ |
| ⋮ | ⋮ |
| $t_n$ | $bs_n$ |

FIG. 5A

| $b_1$ |
|---|
| $b_2$ |
| ⋮ |
| $b_n$ |

HEART RATE INFORMATION
STANDARDIZATION MODEL

| $bs_1$ |
|---|
| $bs_2$ |
| ⋮ |
| $bs_n$ |

FIG. 5B

# FIG. 6

HEART RATE INFORMATION
STANDARDIZATION MODULE

INFOMATION OBTAINED
BY FIRST TYPE
WEARABLE DEVICE → FIRST TYPE MODEL → STANDARDIZED HEART RATE INFORMATION

INFORMATION OBTAINED
BY SECOND TYPE
WEARABLE DEVICE → SECOND TYPE MODEL → STANDARDIZED HEART RATE INFORMATION

FIG. 7

```
                          ┌ ─ ─ ─ ─ ─ ─ ─ ─ ─ ─ ─ ─ ─ ┐
                          │   HEART RATE INFORMATION   │
                          │  STANDARDIZATION MODULE    │
                          │                            │
                          │      ┌───────────┐         │
HEART RATE INFORMATION    │      │   SLEEP   │         │     STANDARDIZED
                      ───────▶   │   MODEL   │   ───────▶    HEART RATE
       IN SLEEP           │      └───────────┘         │     INFORMATION
                          │                            │
                          │                            │
                          │      ┌───────────┐         │
HEART RATE INFORMATION    │      │ NON-SLEEP │         │     STANDARDIZED
                      ───────▶   │   MODEL   │   ───────▶    HEART RATE
    IN NON-SLEEP          │      └───────────┘         │     INFORMATION
                          └ ─ ─ ─ ─ ─ ─ ─ ─ ─ ─ ─ ─ ─ ┘
```

(STANDARDIZED) HEART RATE
INFORMATION FOR
ESTIMATION PERIOD

(STANDARDIZED) HEART RATE
INFORMATION FOR
REFERENCE PERIOD

FIRST DISEASE
MONITORING
MODEL

DISEASE
MONITORING
INFORMATION

## FIG. 8A

(STANDARDIZED) HEART RATE
INFORMATION FOR
ESTIMATION PERIOD

REPRESENTATIVE
HEART RATE FOR
ESTIMATION PERIOD

(STANDARDIZED) HEART RATE
INFORMATION FOR
REFERENCE PERIOD

REPRESENTATIVE
HEART RATE FOR
REFERENCE PERIOD

SECOND DISEASE
MONITORING
MODEL

DISEASE
MONITORING
INFORMATION

## FIG. 8B

FIG. 9

**FIG. 10A**

| $t_1$ | $b_1$ |
| $t_2$ | |
| $t_3$ | $b_3$ |
| ... | ... |
| $t_n$ | $b_n$ |

1

MISSING VALUE ESTIMATION →

| $t_1$ | $b_1$ |
| $t_2$ | $b'_2$ |
| $t_3$ | $b_3$ |
| ... | ... |
| $t_n$ | $b_n$ |

3

HEART RATE INFORMATION STANDARDIZATION MODEL →

| $t_1$ | $bs_1$ |
| $t_2$ | $bs_2$ |
| $t_3$ | $bs_3$ |
| ... | ... |
| $t_n$ | $bs_n$ |

4

**FIG. 10B**

| $t_1$ | $b_1$ | $m_1$ | $d_1$ |
| $t_2$ | | $m_2$ | $d_2$ |
| $t_3$ | $b_3$ | $m_3$ | $d_3$ |
| ... | ... | ... | ... |
| $t_n$ | $b_n$ | $m_n$ | $d_n$ |

HEART RATE INFORMATION STANDARDIZATION MODEL →

| $t_1$ | $bs_1$ |
| $t_2$ | $bs_2$ |
| $t_3$ | $bs_3$ |
| ... | ... |
| $t_n$ | $bs_n$ |

34

# FIG. 11

OBTAINING TYPE INFORMATION OF WEARABLE
DEVICE AND HEART RATE INFORMATION ——S1110

GENERATING STANDARDIZED HEART RATE INFORMATION USING
TYPE INFORMATION AND HEART RATE INFORMATION THROUGH
HEART RATE INFORMATION STANDARDIZATION MODULE ——S1120

GENERATING DISEASE MONITORING INFORMATION USING
STANDARDIZED HEART RATE INFORMATION THROUGH THE
DISEASE MONITORING MODULE ——S1130

OUTPUTTING DISEASE MONITORING INFORMATION ——S1140

FIG. 12

EP 4 497 375 A1

# FIG. 13

100 — WEARABLE DEVICE

200 — USER TERMINAL

500 — DISEASE MONITORING SERVER

GENERATING HEART RATE INFORMATION (S1310)

HEART RATE INFORMATION →

STORING HEART RATE INFORMATION (S1320)

HEART RATE INFORMATION →

MONITORING DISEASE (S1330)

← DISEASE MONITORING INFORMATION

DISPLAYING DISEASE MONITORING INFORMATION

EP 4 497 375 A1

# FIG. 14

EP 4 497 375 A1

## FIG. 15

100 — WEARABLE DEVICE

200 — USER TERMINAL

500 — DISEASE MONITORING SERVER

GENERATING HEART RATE INFORMATION (S1510)

HEART RATE INFORMATION → HEART RATE INFORMATION →

STORING HEART RATE INFORMATION (S1520)

MONITORING DISEASE (S1530)

← DISEASE MONITORING INFORMATION

DISPLAYING DISEASE MONITORING INFORMATION (S1540)

EP 4 497 375 A1

FIG. 16

OBTAINING FIRST USER INFORMATION AND
FIRST TYPE INFORMATION FOR FIRST USER — S1610

OBTAINING SECOND USER INFORMATION AND
SECOND TYPE INFORMATION
FOR SECOND USER — S1620

STORING INFORMATION ABOUT USERS
INCLUDING FIRST USER AND SECOND USER — S1630

FIG. 17

EP 4 497 375 A1

FIG. 18

EP 4 497 375 A1

FIG. 19

FIG. 20

<div align="center">**INTERNATIONAL SEARCH REPORT**</div>

| | International application No. |
|---|---|
| | **PCT/KR2023/003723** |

**A. CLASSIFICATION OF SUBJECT MATTER**

**A61B 5/00**(2006.01)i; **A61B 5/024**(2006.01)i; **G16H 50/20**(2018.01)i

According to International Patent Classification (IPC) or to both national classification and IPC

**B. FIELDS SEARCHED**

Minimum documentation searched (classification system followed by classification symbols)

A61B 5/00(2006.01); A61B 5/0402(2006.01); A61B 5/0452(2006.01); A61B 5/318(2021.01); G01D 3/00(2006.01); G01D 3/028(2006.01); G16H 50/20(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심박 (heart rate), 센서 (sensor), 인공 지능 (artificial intelligence), 학습 (learning), 표준화 (standardization)

**C. DOCUMENTS CONSIDERED TO BE RELEVANT**

| Category* | Citation of document, with indication, where appropriate, of the relevant passages | Relevant to claim No. |
|---|---|---|
| A | JP 2015-112423 A (KYOTO UNIV.) 22 June 2015 (2015-06-22)<br>See paragraphs [0028]-[0035]; and claim 1. | 1-21 |
| A | KR 10-2078703 B1 (LEE, Jae Yong) 19 February 2020 (2020-02-19)<br>See paragraphs [0035]-[0043] and [0090]; and claim 1. | 1-21 |
| A | KR 10-2021-0058274 A (KWON, Joon-Myoung) 24 May 2021 (2021-05-24)<br>See entire document. | 1-21 |
| A | JP 2020-046346 A (NIPPON TELEGR & TELEPH CORP. <NTT>) 26 March 2020 (2020-03-26)<br>See entire document. | 1-21 |
| PX | KR 10-2462245 B1 (THYROSCOPE INC.) 03 November 2022 (2022-11-03)<br>See claims 1-21.<br>※Published patent of a priority application of the present international application. | 1-21 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 June 2023** | **27 June 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/003723**

| Patent document cited in search report | | | Publication date (day/month/year) | Patent family member(s) | | | Publication date (day/month/year) |
|---|---|---|---|---|---|---|---|
| JP | 2015-112423 | A | 22 June 2015 | JP | 6344912 | B2 | 20 June 2018 |
| KR | 10-2078703 | B1 | 19 February 2020 | WO | 2021-015570 | A1 | 28 January 2021 |
| KR | 10-2021-0058274 | A | 24 May 2021 | None | | | |
| JP | 2020-046346 | A | 26 March 2020 | JP | 7068583 | B2 | 17 May 2022 |
| | | | | US | 2021-0357812 | A1 | 18 November 2021 |
| | | | | WO | 2020-059555 | A1 | 26 March 2020 |
| KR | 10-2462245 | B1 | 03 November 2022 | None | | | |

Form PCT/ISA/210 (patent family annex) (July 2022)